(19) Europäisches Patentamt

European Patent Office

Office européen des brevets

(11) **EP 0 726 905 B1**

(12) **EUROPEAN PATENT SPECIFICATION**

(45) Date of publication and mention
of the grant of the patent:
**23.03.2005 Bulletin 2005/12**

(51) Int Cl.[7]: **C07H 21/04**, C12Q 1/68

(21) Application number: **95900520.8**

(22) Date of filing: **02.11.1994**

(86) International application number:
**PCT/US1994/012632**

(87) International publication number:
**WO 1995/012607 (11.05.1995 Gazette 1995/20)**

(54) **SINGLE NUCLEOTIDE POLYMORPHISMS AND THEIR USE IN GENETIC ANALYSIS**

POLYMORPHISMUS VON MONONUKLEOTIDEN UND IHRE VERWENDUNG IN DER
GENANALYSE

POLYMORPHISMES DE MONONUCLEOTIDE ET LEUR UTILISATION EN ANALYSE GENETIQUE

(84) Designated Contracting States:
**AT BE CH DE DK ES FR GB GR IE IT LI LU MC NL
PT SE**

(30) Priority: **03.11.1993 US 145145
23.03.1994 US 216538**

(43) Date of publication of application:
**21.08.1996 Bulletin 1996/34**

(73) Proprietor: **Orchid BioSciences, Inc.
Princeton, NJ 08540 (US)**

(72) Inventors:
• **GOELET, Philip
Cockeysville, MD (US)**
• **KNAPP, Michael, R.
California 95003 (US)**

(74) Representative: **Bentham, Andrew et al
J.A. Kemp & Co.
14 South Square
Gray's Inn
London WC1R 5JJ (GB)**

(56) References cited:
| | |
|---|---|
| **EP-A- 0 012 351** | **EP-A- 0 246 864** |
| **EP-A- 0 370 719** | **EP-A- 0 412 883** |
| **WO-A-89/10414** | **WO-A-89/10977** |
| **WO-A-90/01069** | **WO-A-90/09455** |
| **WO-A-92/10589** | **WO-A-92/15712** |
| **WO-A-92/16657** | |

• **LI; SADLER: 'Low Nucleotide Diversity in Man'
GENETICS vol. 129, October 1991, pages 513 -
523**

**Description**

## FIELD OF THE INVENTION

The present invention is in the field of recombinant DNA technology. More specifically, the invention is directed to methods suitable for identifying single nucleotide polymorphisms in the genome of an animal, especially a horse or a human, and using such sites to analyze identity, ancestry or genetic traits.

## BACKGROUND OF THE INVENTION

The capacity to genotype an animal, plant or microbe is of fundamental importance to forensic science, medicine and epidemiology and public health, and to the breeding and exhibition of animals. Such a capacity is needed, for example, to determine the identity of the causative agent of an infectious disease, to determine whether two individuals are related, or to establish whether a particular animal such as a horse is a thoroughbred.

The analysis of identity and parentage, along with the capacity to diagnose disease is also of central concern to human, animal and plant genetic studies, particularly forensic or paternity evaluations, arid in the evaluation of an individual's risk of genetic disease. Such goals have been pursued by analyzing variations in DNA sequences that distinguish the DNA of one individual from another.

If such a variation alters the lengths of the fragments that are generated by restriction endonuclease cleavage, the variations are referred to as restriction fragment length polymorphisms ("RFLPs"). RFLPs have been widely used in human and animal genetic analyses (Glassberg, J., UK patent Application 2135774; Skolnick, M.H. et al., Cytogen. Cell Genet. 32:58-67 (1982); Botstein, D. et al., Ann. J. Hum. Genet. 32:314-331 (1980); Fischer, S.G et al. (PCT Application WO90/13668); Uhlen, M., PCT Application WO90/11369)). Where a heritable trait can be linked to a particular RFLP, the presence of the RFLP in a target animal can be used to predict the likelihood that the animal will also exhibit the trait. Statistical methods have been developed to permit the multilocus analysis of RFLPs such that complex traits that are dependent upon multiple alleles can be mapped (Lander, S. et al., Proc. Natl. Acad. Sci. (U.S.A.) 83: 7353-7357 (1986); Lander, S. et. al., Proc. Natl. Acad. Sci. (U.S.A.) 84:2363-2367 (1987); Donis-Keller, H. et al., Cell 51:319-337 (1987); Lander, S. et al., Genetics 121:185-199 (1989)). Such methods can be used to develop a genetic map, as well as to develop plants or animals having more desirable traits (Donis-Keller, H. et al., Cell 51:319-337 (1987); Lander, S. et al., Genetics 121:185-199 (1989)).

In some cases, the DNA sequence variations are in regions of the genome that are characterized by short tandem repeats (STRs) that include tandem di- or tri-nucleotide repeated motifs of nucleotides. These tandem repeats are also referred to as "variable number tandem repeat" ("VNTR") polymorphisms. VNTRs have been used in identity and paternity analysis (Weber, J.L., U.S. Patent 5,075,217; Armour, J.A.L. et al., FEBS Lett. 307:113-115 (1992); Jones, L. et al., Eur. J. Haematol. 39:144-147 (1987); Horn, G.T. et al., PCT Application WO91/14003; Jeffreys, A.J., European Patent Application 370,719; Jeffreys, A.J., U.S. Patent 5,175,082); Jeffreys. A.J. et al., Amer. J. Hum. Genet. 39:11-24 (1986); Jeffreys. A.J. et al., Nature 316:76-79 (1985); Gray, I.C. et al., Proc. R. Acad. Soc. Lond. 243:241-253 (1991); Moore, S.S. et al., Genomics 10:654-660 (1991); Jeffreys, A.J. et al., Anim. Genet. 18:1-15 (1987); Hillel, J. et al., Anim. Genet. 20:145-155 (1989); Hillel, J. et al., Genet. 124:783-789 (1990)) and are now being used in a large number of genetic mapping studies.

Li and Sadler (Genetics 129:513-523 (1991)) have studied nucleotide diversity in humans using published cDNA and genomic sequences. Their measure of genetic variability was defined as the number of nucleotide differences per site between two randomly chosen sequences from a population. On this basis, human nucleotide diversity was found to be low.

A third class of DNA sequence variation results from single nucleotide polymorphisms (SNPs) that exist between individuals of the same species. Such polymorphisms are far more frequent than RFLPs, STRs and VNTRs. In some cases, such polymorphisms comprise mutations that are the determinative characteristic in a genetic disease. Indeed, such mutations may affect a single nucleotide in a protein-encoding gene in a manner sufficient to actually cause the disease (i.e. hemophilia, sickle-cell anemia, etc.). In many cases, these SNPs are in noncoding regions of a genome. Despite the central importance of such polymorphisms in modern genetics, no practical method has been developed that permits the use of highly parallel analysis of many SNP alleles in two or more individuals in genetic analysis.

The present invention provides such an improved method. Indeed, the present invention provides methods and gene sequences that permit the genetic analysis of identity and parentage, and the diagnosis of disease by discerning the variation of single nucleotide polymorphisms.

## SUMMARY OF THE INVENTION

The present invention is directed to molecules that. comprise single nucleotide polymorphisms (SNPs) that are present in mammalian DNA, and in particular, to equine and human genomic DNA polymorphisms. The invention is directed to methods for (i) identifying novel, single nucleotide polymorphisms (ii) methods for the repeated, analysis and testing of these SNPs in different samples and (iii) methods for exploiting the existence of such sites in the genetic analysis of single animate and populations of animals.

The analysis (genotyping) of such sites is useful in determining identity, ancestry, predisposition to genetic disease, the presence or absence of a desired trait, etc. In detail, the invention provides:

a method of genetic analysis of a set of individuals of the same species comprising:

providing a polymorphic array comprising a set of single nucleotide polymorphisms (SNPs); and
determining the presence or absence of the polymorphisms in the set of SNPs in each of the set of individuals; and
determining whether the presence or absence of a particular allele of a polymorphism in the set of SNPs is associated with a particular trait.

The invention also provides:

a method of determining the probability that a nucleic acid sample is derived from a particular individual comprising:

providing a polymorphic array comprising a set of single nucleotide polymorphisms (SNPs) from said individual and a corresponding polymorphic array from said sample;
determining the presence or absence of multiple SNP markers in the two arrays and comparing the results for each SNP marker;
determining therefrom a probability of identity or non-identity from each comparison; and
determining therefrom a cumulative probability of identity or non-identity by multiplying the probabilities provided by each comparison.

The invention also provides:

a method of determining the likelihood that an individual is or is not the progeny of a putative ancestor or ancestors comprising:

providing a polymorphic array comprising a set of single nucleotide polymorphisms (SNPs) from said individual and a corresponding polymorphic array from said putative ancestor or ancestors;
determining the presence or absence of multiple SNP markers in the individual array and the putative ancestor's or ancestors' and comparing the results for each SNP marker; and
determining therefrom the likelihood that the individual is or is not the progeny of the putative ancestor or ancestors.

The invention also provides:

a method of generating a genetic map of an individual, comprising:

(a) providing a polymorphic array comprising three or more single nucleotide polymorphisms (SNPs);
(b) identifying the SNP variants present in an ancestor of the individual by determining the base identity at each SNP site of the ancestor of the individual and identifying the SNP variants present in the individual by determining the base identity at each SNP site of the individual;
(c) determining the number of matches between the individual and the ancestor,
(d) calculating the extent of genetic linkage between each allele from the number of matches of step (c) and the probability that any pair of alleles found in the individual were inherited from the same ancestor based on the allelic frequencies of the SNP variants of the polymorphic array, thereby generating the genetic map of the individual.

**BRIEF DESCRIPTION OF THE FIGURES**

Figure 1 illustrates the preferred method for cloning random genomic fragments. Genomic DNA us size fractionated, and then introduced into a plasmid vector, in order to obtain random clones. PCR primers are designed, and used to sequence the inserted genomic sequences.

Figure 2 illustrates the data generated by preferred method for identifying new polymorphic sequences which is cycle sequencing of a random genomic fragment.

Figure 3 illustrates the RFLP method for screening random clones for polymorphic sequences. After the initial optimization of PCR conditions (top panel), amplified material is cleaved with several restriction enzymes, and the resulting profiles are analyzed (middle panels). A population study is then performed to determine allelic frequencies.

Figure 4 shows a graph of the probability that two individuals will have identical genotypes with given panels of genetic markers. The number of tests employed is plotted on the abscissa while the cumulative probability of non-identity is plotted on the ordinate. The horizontal line indicates 0.95 probability of non-identity. Legend: o indicates the extrapolated prototype; x indicates 3 alleles (51%, 34%, 15%); triangle indicates 2 alleles (79%, 21%).

Figure 5 shows a graph of the probability that given panels of 20 genetic markers will exclude a random alleged father in a paternity suit in which the mother is not in question. The number of tests employed is plotted on the abscissa while the cumulative probability of exclusion is plotted on the ordinate. The horizontal line indicates 0.95 probability of exclusion. The legend is as in Figure 4.

Figure 6 uses the SNP identified in clone 177-2 to illustrate the organization of the sequences in Table 1.

Figure 7 illustrates the preferred method for genotyping SNPs. The seven steps illustrate how GBA can be performed starting with a biological sample.

Figures 8A and 8B illustrate how horse parentage data appears at the microtiter plate level.

**DESCRIPTION OF THE PREFERRED EMBODIMENTS**

**I. The Single Nucleotide Polymorphisms of the Present Invention and The Advantages of their Use in Genetic Analysis**

**A. The Attributes of the Polymorphisms**

The particular gene sequences of interest to the present invention comprise "single nucleotide polymorphisms." A "polymorphism" is a variation in the DNA sequence of some members of a species. The genomes of animals and plants naturally undergo spontaneous mutation in the course of their continuing evolution (Gusella, J.F., Ann. Rev. Biochem. 55:831-854 (1986)). The majority of such mutations create polymorphisms. The mutated sequence and the initial sequence co-exist in the species' population. In some instances, such coexistence is in stable or quasi-stable equilibrium. In other instances, the mutation confers a survival or evolutionary advantage to the species, and accordingly, it may eventually (i.e. over evolutionary time) be incorporated into the DNA of every member of that species.

A polymorphism is thus said to be "allelic," in that, due to the existence of the polymorphism, some members of a species may have the unmutated sequence (i.e. the original "allele") whereas other members may have a mutated sequence (i.e. the variant or mutant "allele"). In the simplest case, only one mutated sequence may exist, and the polymorphism is said to be diallelic. Diallelic polymorphisms are the most common and the preferred polymorphisms of the present invention. The occurrence of alternative mutations can give rise to trialleleic, etc. polymorphisms. An allele may be referred to by the nucleotide(s) that comprise the mutation. Thus, for example, in Table 1, clone 177-2 (SEQ ID NO:1 and SEQ ID NO:2) illustrates the sequence of one strand of a diallelic polymorphism in which one allele has a "C" and the other allele has a "T" at the polymorphic site.

The present invention is directed to a particular class of allelic polymorphisms, and to their use in genotyping a plant or animal. Such allelic polymorphisms are referred to herein as "single nucleotide polymorphisms," or "SNPs." "Single nucleotide polymorphisms" are defined by the following attributes. A central attribute of such a polymorphism is that it contains a polymorphic site, "X," most preferably occupied by a single nucleotide, which is the site of variation between allelic sequences. A second characteristic of an SNP is that its polymorphic site "X" is preferably preceded by and followed by "invariant" sequences of the allele. The polymorphic site of the SNP is thus said to lie "immediately" 3' to a "5'-proximal" invariant sequence, and "immediately" 5' to a "3'-distal" invariant sequence. Such sequences flank the polymorphic site.

As used herein, a sequence is said to be an "invariant" sequence of an allele if the sequence does not vary in the population of the species, and if mapped, would map to a "corresponding" sequence of the same allele in the genome of every member of the species population. Two sequences are said to be "corresponding" sequences if they are analogs of one another obtained from different sources. The gene sequences that encode hemoglobin in two humans illustrate "corresponding" allelic sequences. The definition of "corresponding alleles" provided herein is intended to clarify, but not to alter, the meaning of that term as understood by those of ordinary skill in the art. Each row of Table 1 shows the identity of the nucleotide of the polymorphic site of "corresponding" equine alleles, as well as the invariant 5'-proximal and 3'-distal sequences that are also attributes of that SNP. "Correspondiong alleles" are illustrated in Table 5 with regard to human alleles. Each row of Table 5 shows the identity of the nucleotide of the polymorphic site of "corresponding" human alleles, as well as the invariant 5'-proximal and 3'-distal sequences that are also attributes of that SNP.

Since genomic DNA is double-stranded, each SNP can be defined in terms of either strand. Thus, for every SNP, one strand will contain an immediately 5'-proximal invariant sequence and the other will contain an immediately 3'-distal invariant sequence. In the preferred embodiment, wherein a SNP's polymorphic site, "X," is a single nucleotide, each strand of the double-stranded DNA of the SNP will contain both an immediately 5'-proximal invariant sequence and an immediately 3'-distal invariant sequence.

Although the preferred SNPs of the present invention involve a substitution of one nucleotide for another at the SNP's polymorphic site, SNPs can also be more complex, and may comprise a deletion of a nucleotide from, or an insertion of a nucleotide into, one of two corresponding sequences. For example, a particular gene sequence may contain an A in a particular polymorphic site in some animals, whereas in other animals a single or multiple base deletion might be present at that site. Although the preferred SNPs of the present invention have both an invariant proximal sequence and invariant distal sequence, SNPs may have only an invariant proximal or only an invariant distal sequence.

Nucleic acid molecules having the a sequence complementary to that of an immediately 3'-distal invariant sequence of a SNP can, if extended in a "template-dependent" manner, form an extension product that would contain the SNP's polymorphic site. An preferred example of such a nucleic acid molecule is a nucleic acid molecule whose sequence is the same as that of a 5'-proximal invariant sequence of the SNP. "Template-dependent" extension refers to the capacity of a polymerase to mediate the extension of a primer such that the extended sequence is complementary to the sequence of a nucleic acid template. A "primer" is a single-stranded oligonucleotide or a single-stranded polynucleotide that is capable of being extended by the covalent addition of a nucleotide in a "template-dependent" extension reaction, In order to possess such a capability, the primer must have a 3'-hydroxyl terminus, and be hybridized to a second nucleic acid molecule (i.e. the "template"). A primer is typically 11 bases or longer; most preferably, a primer is 20 bases, however, primers of shorter or greater length may suffice. A "polymerase" is an enzyme that is capable of incorporating nucleoside triphosphates to extend a 3'-hydroxyl group of a nucleic acid molecule, if that molecule has hybridized to a suitable template nucleic acid molecule. Polymerase enzymes are discussed in Watson, J.D., In: Molecular Biology of the Gene, 3rd Ed., W.A. Benjamin, Inc., Menlo Park, CA (1977)) and similar texts. Other polymerases such as the large proteolytic fragment of the DNA polymerase I of the bacterium E. coli, commonly known as "Klenow" polymerase, E. coli DNA polymerase I, and bacteriophage T7 DNA polymerase, may also be used to perform the method described herein. Nucleic acids having the same sequence as that of the immediately 3' distal invariant sequence of a SNP can be ligated in a template dependent fashion to a primer that has the same sequence as that of the immediately 5' proximal sequence that has been extended by one nucleotide in a template dependent fashion.

## B. The Advantages of Using SNPs in Genetic Analysis

The single nucleotide polymorphic sites of the present invention can be used to analyze the DNA of any plant or animal. Such sites are particularly suitable for analyzing the genome of mammals, including humans, non-human primates, domestic animals (such as dogs, cats, etc.), farm animals (such as cattle, sheep, etc.) and other economically important animals, in particular, horses. They may, however be used with regard to other types of animals, particularly birds (such as chickens, turkeys, etc.) SNPs have several salient advantages over RFLPs, STRs and VNTRs.

First, SNPs occur at greater frequency (approximately 10-100 fold greater), and with greater uniformly than RFLPs and VNTRs. The greater frequency of SNPs means that they can be more readily identified than the other classes of polymorphisms. The greater uniformity of their distribution permits the identification of SNPs "nearer" to a particular trait of interest. The combined effect of these two attributes makes SNPs extremely valuable. For example, if a particular trait (e.g. predisposition to cancer) reflects a mutation at a particular locus, then any polymorphism that is linked to the particular locus can be used to predict the probability that an individual will be exhibiting that trait.

The value of such a prediction is determined in part by the distance between the polymorphism and the locus. Thus, if the locus is located far from any repeated tandem nucleotide sequence motifs, VNTR analysis will be of very limited value. Similarly, if the locus is far from any detectable RFLP, an RFLP analysis would not be accurate. However, since the SNPs of the present invention are present approximately once every 300 bases in the mammalian genome, and exhibit uniformity of distribution, a SNP can, statistically, be found within 150 bases of any particular genetic lesion or mutation. Indeed, the particular mutation may itself be an SNP. Thus, where such locus has been sequenced, the variation in that locus' nucleotide is determinative of the trait in question.

Second, SNPs are more stable than other classes of polymorphisms. Their spontaneous mutation rate is approximately $10^{-9}$, approximately 1,000 times less frequent than VNTRs. Significantly, VNTR-type polymorphisms are characterized by high mutation rates.

Third, SNPs have the further advantage that their allelic frequency can be inferred from the study of relatively few representative samples. These attributes of SNPs permit a much higher degree of genetic resolution of identity, paternity exclusion, and analysis of an animal's predisposition for a particular genetic trait than is possible with either RFLP or VNTR polymorphisms.

Fourth, SNPs reflect the highest possible definition of genetic information -- nucleotide position and base identity. Despite providing such a high degree of definition, SNPs can be detected more readily than either RFLPs or VNTRs, and with greater flexibility. Indeed, because DNA is double-stranded, the complimentary strand of the allele can be analyzed to confirm the presence and identity of any SNP.

The flexibility with which an identified SNP can be characterized is a salient feature of SNPs. VNTR-type polymorphisms, for example, are most easily detected through size fractionation methods that can discern a variation in the number of the repeats. RFLPs are most easily detected by size fractionation methods following restriction digestion.

In contrast, SNPs can be characterized using any of a variety of methods. Such methods include the direct or indirect sequencing of the site, the use of restriction enzymes where the respective alleles of the site create or destroy a restriction site, the use of allele-specific hybridization probes, the use of antibodies that are specific for the proteins encoded by the different alleles of the polymorphism, or by other biochemical interpretation.

The "Genetic Bit Analysis ("GBA") method disclosed by Goelet, P. et al. (WO 92/15712), and discussed below, is a preferred method for detecting the single nucleotide polymorphisms of the present invention. GBA is a method of polymorphic site interrogation in which the nucleotide sequence information surrounding the site of variation in a target DNA sequence is used to design an oligonucleotide primer that is complementary to the region immediately adjacent to, but not including, the variable nucleotide in the target DNA. The target DNA template is selected from the biological sample and hybridized to the interrogating primer. This primer is extended by a single labeled dideoxynucleotide using DNA polymerase in the presence of two, and preferably all four chain terminating nucleoside triphosphate precursors. Cohen, D. et al. (PCT Application WO91/02087) describes a related method of genotyping.

Recently, several primer-guided. nucleotide incorporation procedures for assaying polymorphic sites in DNA have been described (Komher, J. S. et al., Nucl. Acids. Res. 17: 7779-7784 (1989); Sokolov, B. P., Nucl. Acids Res: 18:3671 (1990); Syvänen, A.-C., et al., Genomics 8:684 - 692 (1990); Kuppuswamy, M.N.et al., Proc. Natl. Acad.Sci. (U.S.A.) 88:1143-1147 (1991); Prezant, T.R. et al., Hum. Mutat. 1:159-164 (1992); Ugozzoli, L. et al., GATA 9:107-112 (1992); Nyren,. P. et al., Anal. Biochem. 208:171-175 (1993)). These methods differ from GBA in that they all rely on the incorporation of labeled deoxynucleotides to discriminate between bases at a polymorphic site. In such a format, since the signal is proportional to the number of deoxynucleotides incorporated, polymorphisms that occur in runs of the same nucleotide can result in signals that are proportional to the length of the run (Syvänen, A.-C., et al., Amer. J. Hum. Genet, 52:46-59 (1993)). Such a range of locus-specific signals could be more complex to interpret, especially for heterozygotes, compared to the simple, ternary (2:0, 1:1, or 0:2) class of signals produced by the GBA method. In addition, for some loci, incorporation of an incorrect deoxynucleotide can occur even in the presence of the correct dideoxynucleotide (Komher, J. S. et al., Nucl. Acids. Res. 17:7779-7784 (1989)). Such deoxynucleotide misincorporation events may be due to the Km of the DNA polymerase for the mispaired deoxy- substrate being comparable, in some sequence contexts, to the relatively poor Km of even a correctly base paired dideoxy- substrate (Kornberg, A., et al., In: DNA Replication, 2nd Edition, W.H. Freeman and Co., (1992); New York; Tabor, S. et al., Proc, Natl. Acad. Sci. (U.S.A) 86:4076-4080 (1989)). This effect would contribute to the background noise in the polymorphic site interrogation.

## II. Methods for Discovering Novel Polymorphic Sites

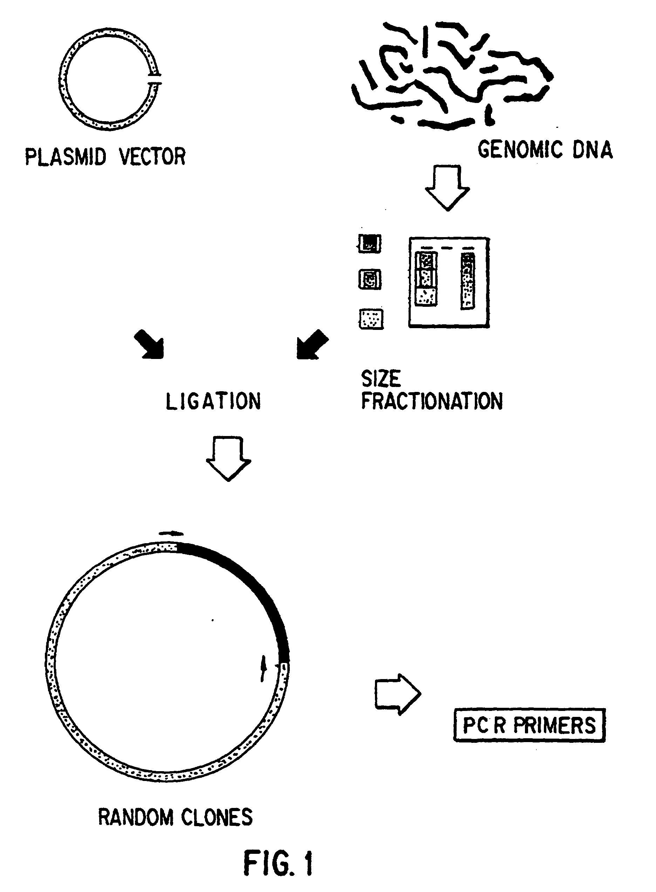

A preferred method for discovering polymorphic sites involves comparative sequencing of genomic DNA fragments from a number of haploid genomes. In the preferred embodiment, illustrated in Figure 1, such sequencing is performed by preparing a random genomic library that contains 0.5-3 kb fragments of DNA derived from one member of a species. Sequences of these recombinants are then used to facilitate PCR sequencing of a number of randomly selected individuals of that species at the same genomic loci.

From such genomic libraries (typically of approximately 50,000 clones), several hundred (200-500) individual clones are purified, and the sequences of the termini of their inserts are determined. Only a small amount of terminal sequence data (100-200 bases) need be obtained to permit PCR amplification of the cloned region. The purpose of the sequencing is to obtain enough sequence information to permit the synthesis of primers suitable for mediating the amplification of the equivalent fragments from genomic DNA samples of other members of the species. Preferably, such sequence determinations are performed using cycle sequencing methodology.

The primers are used to amplify DNA from a panel of randomly selected members of the target species. The number of members in the panel determines the lowest frequency of the polymorphisms that are to be isolated. Thus, if six members are evaluated, a polymorphism that exists at a frequency of, for example, 0.01 might not be identified. In an illustrative, but oversimplified, mathematical treatment, a sampling of six members would be expected to identify only those polymorphisms that occur at a frequency of greater than about .08 (i.e. 1.0 total frequency divided by 6 members divided by 2 alleles per genome). Thus, if one desires the identification of less frequent polymorphisms, a greater number of panel members must be evaluated.

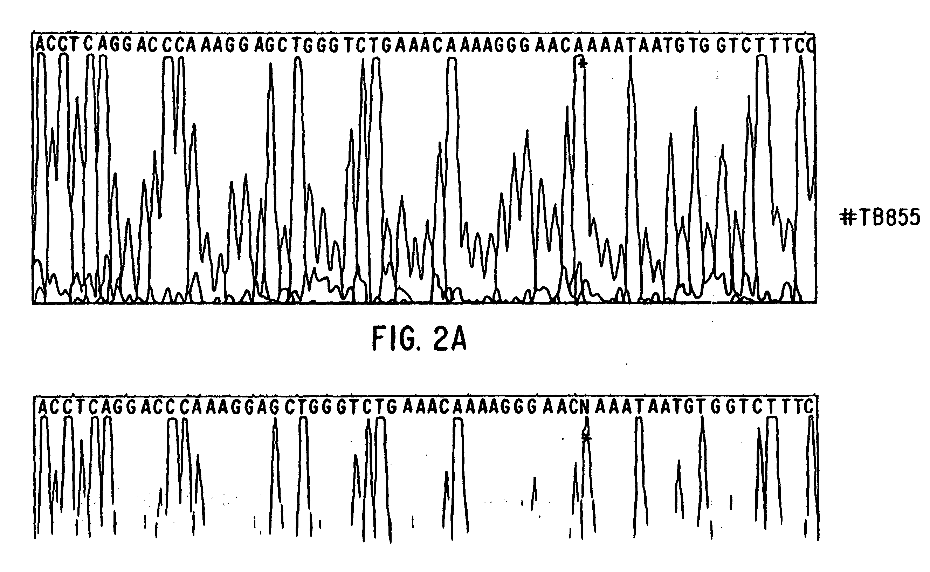

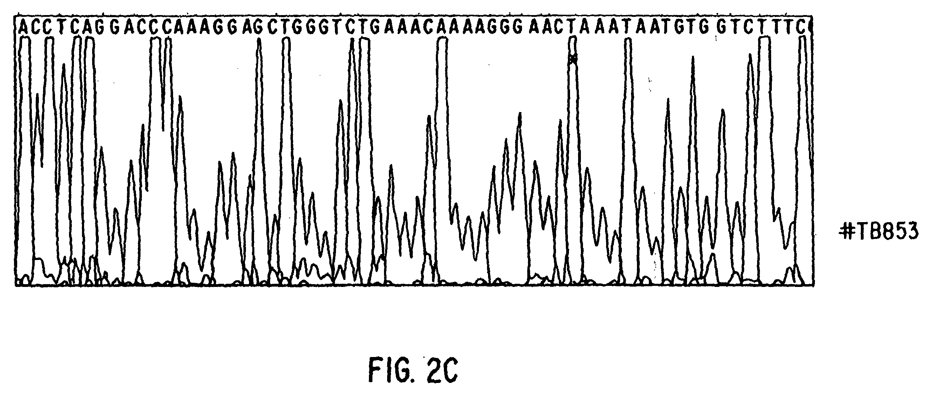

Cycle sequence analysis (Mullis, K. et al., Cold Spring Harbor Symp. Quant. Biol. 51:263-273 (1986); Erlich H. et al., European Patent Appln. 50,424; European Patent Appln. 84,796, European Patent Application 258,017, European Patent Appln. 237,362; Mullis, K., European Patent Appln. 201,184; Mullis K. et al., U.S. Patent No. 4,683,202; Erlich, H., U.S. Patent No. 4,582,788; and Saiki, R. et al., U.S. Patent No. 4,683,194)) is facilitated through the use of automated DNA sequencing instruments and software (Applied Biosystems, Inc.). Differences between sequences of different animals can thereby be identified and confirmed by inspecting the relevant portion of the chromatograms on the computer screen. Differences are interpreted to reflect a DNA polymorphism only if the data was available for both strands, and present in more than one haploid example among the population of animals tested. Figure 2 illustrates the preferred method for identifying new polymorphic sequences which is cycle sequencing of a random genomic fragment. The PCR fragments from five unrelated horses were electroeluted from acrylamide gels and sequenced using repetitive cycles of thermostable Taq DNA polymerase in the presence of a mixture of dNTPs and fluorescent ddNTPs. The products were then separated and analyzed using an automated DNA sequencing instrument of Applied Biosystems, Inc. The data was analyzed using ABI software. Differences between sequences of different animals were identified by the software and confirmed by inspecting the relevant portion of the chromatograms on the computer screen. Differences are presented as "DNA Polymorphisms" only if the data is available for both strands and present in more than one haploid example among the five horses tested. The top panel shows an "A" homozygote, the middle panel an "AT" heterozygote and the bottom panel a "T" homozygote.

Despite the randomized nature of such a search for polymorphisms, such sequencing and comparison of random DNA clones is readily able to identify suitable polymorphisms. Indeed, with respect to the horse, approximately 1/400 nucleotides sequenced by these methods would be discovered as the polymorphic site of an SNP.

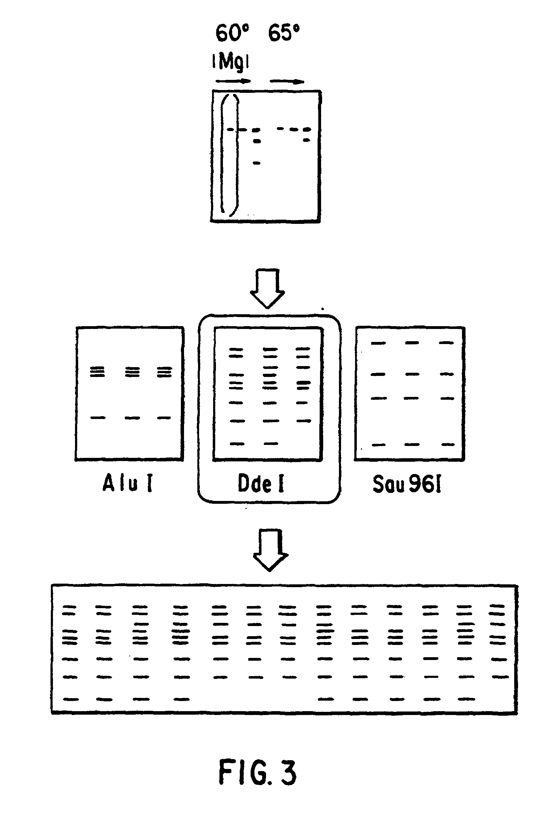

The discovery of polymorphic sites can alternatively be conducted using the strategy outlined in Figure 3. In this embodiment, the DNA sequence polymorphisms are identified by comparing the restriction endonuclease cleavage profiles generated by a panel of several restriction enzymes on products of the PCR reaction from the genomic templates of unrelated members. Most preferably, each of the restriction endonucleases used will have four base recognition sequences, and will therefore allow a desirable number of cuts in the amplified products.

The restriction digestion patterns obtained from the genomic DNAs are preferably compared directly to the patterns obtained from PCR products generated using the corresponding plasmid templates. Such a comparison provides an internal control which indicates that the amplified sequences from the genomic and plasmid DNAs derive from equivalent loci. This control also allows identification of primers that fortuitously amplify repeated sequences, or multicopy loci, since these will generate many more fragments from the genomic DNA templates than from the plasmid templates.

### III. Methods for Genotyping the Single Nucleotide Polymorphisms of the Present Invention

Any of a variety of methods can be used to identify the polymorphic site, "X," of a single nucleotide polymorphism of the present invention. The preferred method of such identification involves directly ascertaining the sequence of the polymorphic site for each polymorphism being analyzed. This approach is thus markedly different from the RFLP method which analyzes patterns of bands rather than the specific sequence of a polymorphism.

### A. Sampling Methods

Nucleic acid specimens may be obtained from an individual of the species that is to be analyzed using either "invasive" or "non-invasive" sampling means. A sampling means is said to be "invasive" if it involves the collection of nucleic acids from within the skin or organs of an animal (including, especially, a murine, a human, an ovine, an equine, a bovine, a porcine, a canine, or a feline animal). Examples of invasive methods include blood collection, semen collection, needle biopsy, pleural aspiration, etc. Examples of such methods are discussed by Kim, C.H. et al. (J. Virol. 66:3879-3882 (1992)); Biswas, B. et al. (Annals NY Acad. Sci. 590:582-583 (1990)); Biswas, B. et al. (J. Clin. Microblol. 29:2228-2233 (1991)).

In contrast, a "non-invasive" sampling means is one in which the nucleic acid molecules are recovered from an internal or external surface of the animal. Examples of such "non-invasive" sampling means include "swabbing," collection of tears, saliva, urine, fecal material, sweat or perspiration, etc. As used herein, "swabbing" denotes contacting an applicator/collector ("swab") containing or comprising an adsorbent material to a surface in a manner sufficient to collect surface debris and/or dead or sloughed off cells or cellular debris. Such collection may be accomplished by swabbing nasal, oral, rectal, vaginal or aural orifices, by contacting the skin or tear ducts, by collecting hair follicles, etc.

Nasal swabs have been used to obtain clinical specimens for PCR amplification (Olive, D.M. et al., J. Gen. Virol. 71:2141-2147 (1990); Wheeler, J.G. et al., Amer. J. Vet. Res. 52:1799-1803 (1991)). The use of hair follicles to identify VNTR polymorphisms for paternity testing in horses has been described by Ellegren, H. et al. (Animal Genetics 23:133-142 (1992). The reference states that a standardized testing system based on PCR-analyzed microsatellite polymorphisms are likely to be an alternative to blood typing for paternity testing.

A preferred swab for the collection of DNA will comprise a solid support, at least a portion of which is designed to adsorb DNA. The portion designed to adsorb DNA may be of a compressible texture, such as a "foam rubber," or the like. Alternatively, it may be an adsorptive fibrous composition, such as cotton, polyester, nylon, or the like. In yet another embodiment, the portion designed to adsorb DNA may be an abrasive material, such as a bristle or brush, or having a rough surface. The portion of the swab that is designed to adsorb DNA may be a combination of the above textures and compositions (such as a compressible brush, etc.). The swab will, preferably, be specially formed in a substantially rod-like, arrow-like or mushroom-like shape, such that it will have a segment that can be held by the collecting individual, and a tip or end portion which can be placed into contact with the surface that contains the sample DNA that is to be collected. In one embodiment, the swab will be provided with a storage chamber, such as a plastic or glass tube or cylinder, which may have one open end, such as a test-tube. Alternatively, the tube may have two open ends, such that after swabbing, the collector can pull on one end of the swab so as to cause the other end of the swab to be withdrawn into the tube. In yet another embodiment, the tube may have two open ends, such that after swabbing, the tube can be converted into a column to assist in the further processing of the collected DNA. In one embodiment, the end or ends of the storage chamber are self-sealing after swabbing has been accomplished.

The swab or the storage chamber may contain antimicrobial agents at concentrations sufficient to prevent the proliferation of microbes (bacteria, yeast, molds, etc.) during subsequent storage or handling.

In one embodiment, the swab or storage chamber will contain an chromogenic reagent which reacts to the presence of DNA to yield a detectable signal that can be identified at the time of sample collection. Most preferably, such a reagent will comprise a minimum concentration "open-end point" assay for DNA. Such an assay is capable of detecting concentrations of nucleic acids that range from the minimum detection level of the assay to the maximum assay saturation level of the assay. This saturation level is adjustable, and can be increased by decreasing the time of reaction. Preferred chromogenic reagents include anti-DNA antibodies that are conjugated to enzymes, diaminopimelic acid, etc.

## B. Amplification-Based Analysis

The detection of polymorphic sites in a sample of DNA may be facilitated through the use of DNA amplification methods. Such methods specifically increase the concentration of sequences that span the polymorphic site, or include that site and sequences located either distal or proximal to it. Such amplified molecules can be readily detected by gel electrophoresis or other means.

The most preferred method of achieving such amplification employs PCR, using primer pairs that are capable of hybridizing to the proximal sequences that define a polymorphism in its double-stranded form.

in lieu of PCR, alternative methods, such as the "Ligase Chain Reaction" ("LCR") may be used (Barany, F., Proc. Natl. Acad. Sci. (U.S.A.) 88:189-193 (1991). LCR uses two pairs of oligonucleotide probes to exponentially amplify a specific target. The sequences of each pair of oligonucleotides is selected to permit the pair to hybridize to abutting sequences of the same strand of the target. Such hybridization forms a substrate for a template-dependent ligase. As with PCR, the resulting products thus serve as a template in subsequent cycles and an exponential amplification of the desired sequence is obtained.

In accordance with the present invention, LCR can be performed with oligonucleotides having the proximal and distal sequences of the same strand of a polymorphic site. In one embodiment, either oligonucleotide will be designed to include the actual polymorphic site of the polymorphism. In such an embodiment, the reaction conditions are selected such that the oligonucleotides can be ligated together only if the target molecule either contains or lacks the specific nucleotide that is complementary to the polymorphic site present on the oligonucleotide.

In an alternative embodiment, the oligonucleotides will not include the polymorphic site, such that when they hybridize to the target molecule, a "gap" is created (see, Segev, D., PCT Application WO 90/01069). This gap is then "filled" with complementary dNTPs (as mediated by DNA polymerase), or by an additional pair of oligonucleotides. Thus, at the end of each cycle, each single strand has a complement capable of serving as a target during the next cycle and exponential amplification of the desired sequence is obtained.

The "Oligonucleotide Ligation Assay" ("OLA") (Landegren, U. et al., Science 241:1077-1080 (1988)) shares certain similarities with LCR and may also be adapted for use in polymorphic analysis. The OLA protocol uses two oligonucleotides which are designed to be capable of hybridizing to abutting sequences of a single strand of a target. OLA, like LCR, is particularly suited for the detection of point mutations. Unlike LCR, however, OLA results in "linear" rather than exponential amplification of the target sequence.

Nickerson, D.A. et al. have described a nucleic acid detection assay that combines attributes of PCR and OLA (Nickerson, D.A. et al., Proc. Natl. Acad. Sci. (U.S.A.) 87:8923-8927 (1990). In this method, PCR is used to achieve the exponential amplification of target DNA, which is then detected using OLA. In addition to requiring multiple, and separate, processing steps, one problem associated with such combinations is that they inherit all of the problems associated with PCR and OLA.

Schemes based on ligation of two (or more) oligonucleotides in the presence of nucleic acid having the sequence of the resulting "di-oligonucleotide", thereby amplifying the di-oligonucleotide, are also known (Wu, D.Y. et al., Genomics 4:560 (1989)), and may be readily adapted to the purposes of the present invention.

Other known nucleic acid amplification procedures, such as transcription-based amplification systems (Malek, L.T. et al., U.S. Patent 5,130,238; Davey, C. et al., European Patent Application 329,822; Schuster et al., U.S. Patent 5,169,766; Miller, H.I. et al., PCT appln. WO 89/06700; Kwoh, D. et al., Proc. Natl. Acad. Sci. (U.S.A.) 86:1173 (1989); Gingeras, T.R. et al., PCT application WO 88/10315)), or isothermal amplification methods (Walker, G.T. et al., Proc. Natl. Acad. Sci. (U.S.A.) 89:392-396 (1992)) may also be used.

### C. Preparation of Single-Stranded DNA

The direct analysis of the sequence of an SNP of the present invention can be accomplished using either the "dideoxy-mediated chain termination method," also known as the "Sanger Method" (Sanger, F., et al., J. Molec. Biol. 94:441 (1975)) or the "chemical degradation method," "also known as the "Maxam-Gilbert method" (Maxam, A.M., et al., Proc. Natl. Acad. Sci. (U.S.A.) 74:560 (1977), both references herein incorporated by reference). Methods for sequencing DNA using either the dideoxy-mediated method or the Maxam-Gilbert method are widely known to those of ordinary skill in the art. Such methods are, for example, disclosed in Sambrook, J., et al., Molecular Cloning, a Laboratory Manual, 2nd Edition, Cold Spring Harbor Press, Cold Spring Harbor, New York (1989), and in Zyskind, J. W., et al., Recombinant DNA Laboratory Manual, Academic Press. Inc., New York (1988).

Where a nucleic acid sample contains double-stranded DNA (or RNA), or where a double-stranded nucleic acid amplification protocol (such as PCR) has been employed, it is generally desirable to conduct such sequence analysis after treating the double-stranded molecules so as to obtain a preparation that is enriched for, and preferably predominantly, only one of the two strands.

The simplest method for generating single-stranded DNA molecules from double-stranded DNA is denaturation using heat or alkalai treatment.

Single-stranded DNA molecules may also be produced using the single-stranded DNA bacteribphage M13 (Messing, J. et al., Meth. Enzymol. 101:20 (1983); see also, Sambrook, J., et al. (In: Molecular Cloning: A Laboratory Manual, Cold Spring Harbor Laboratory Press, Cold Spring, Harbor. NY (1989)).

Several alternative methods can be used to generate single-stranded DNA molecules. Gyllensten, U. et al., (Proc. Natl. Acad. Sci. (U.S.A.) 85:7652-7656 (1988) and Mihovilovic, M. et al., (BioTechniques 7(1):14 (1989)) describe a method, termed "asymmetric PCR," in which the standard "PCR" method is conducted using primers that are present in different molar-concentrations. Higuchi, R.G. et al. (Nucleic Acids Res. 17:5865 (1985)) exemplifies an additional method for generating single-stranded amplification products. The method entails phosphorylating the 5'-terminus of one strand of a double-stranded amplification product, and then permitting a 5' → 3' exonuclease (such as exonuclease) to preferentially degrade the phosphorylated strand.

Other methods, have also exploited the nuclease resistant properties of phosphorothioate derivatives in order to generate single-stranded DNA molecules (Benkovic et al., U.S. Patent No. 4,521,509; June 4, 1985); Sayers, J.R. et al. (Nucl. Acids Res. 16:791-802 (1988); Eckstein F. et al., Biochemistry 15:1685-1691 (1976); Ott, J., et al., Biochemistry 26:8237-8241 (1987)).

A discussion of the relative advantages and disadvantages. of such methods of producing single-stranded molecules is provided by Nikiforov, T. (U.S. patent application serial no. 08/005,061, corresponding to WO94/16090. published 21 July 1994).

Most preferably, such single-stranded molecules will be produced using the methods described by Nikiforov, T. (Supra). In brief, these methods employ nuclease resistant nucleotides derivatives, and incorporates such derivatives, by chemical synthesis or enzymatic means, into primer molecules, or their extension products, in place of naturally occurring nucleotides.

Suitable nucleotide derivatives include derivatives in which one or two of the non-bridging oxygens of the phosphate moiety of a nucleotide has been replaced with a sulfur-containing group (especially a phosphorothioate), an alkyl group (especially a methyl or ethyl alkyl group), a nitrogen-containing group (especially an amine), and/or a selenium-containing group, etc.

Phosphorothioate deoxyribonucleotide or ribonucleotide derivatives (e.g. a nucleoside 5'-O-1-thiotriphosphate) are the most preferred nucleotide derivatives. Any of a variety of chemical methods may be used to produce such phosphorothioate derivatives (see, for example, Zon, G. et al., Anti-Canc. Drug Des. 6:539-568 (1991); Kim, S. G. et al., Biochem. Biophys. Res. Commun. 179:1-614-1619 (1991); Vu, H. et al., Tetrahedron Lett. 32:3005-3008 (1991); Taylor, J.W. et al., Nucl. Acids Res. 13:8749-8764 (1985); Eckstein, F. et al., Biochemistry 15:1685-1691 (1976); Ott, J. et al., Biochemistry 26:8237-8241 (1987); Ludwig, J. et al., J. Org. Chem. 54:631-635, (1989)). Phosphorothioate nucleotide derivatives can also be obtained commercially from Amersham or Pharmacia.

Importantly, the selected nucleotide derivative must be suitable for in vitro primer-mediated extension and provide nuclease resistance to the region of the nucleic acid molecule in which it is incorporated. In the most preferred embodiment, it must confer resistance to exonucleases that attack double-stranded DNA from the 5'-end (5'→3' exonucleases). Examples of such exonucleases include bacteriophage T7 gene 6 exonuclease ("T7 exonuclease) and the bacteriophage lambda exonuclease ("λ exonuclease"). Both T7 exonuclease and λ exonuclease are inhibited to a significant degree by the presence of phosphorothioate, bonds so as to allow the selective degradation of one of the strands. However, any double-strand specific. 5'→3' exonuclease can be used for this process, provided that its activity is affected by the presence of the bonds of the nuclease resistant nucleotide derivatives. The preferred enzyme when using phosphorothioate derivatives is the T7 gene 6 exonuclease, which shows maximal enzymatic activity in the same buffer used for many DNA dependent polymerase buffers including Taq polymerase. The 5'→3' exonuclease resistant properties of phosphorothioate derivative-containing DNA molecules are discussed, for example, in Kunkel, T.A. (In: Nucleic Acids and Molecular Biology, Vol. 2, 124-135 (Eckstein, F. et al., eds.), Springer-Verlag, Berlin, (1988)). The 3'→5' exonuclease resistant properties of phosphorothioate nucleotide containing nucleic acid molecules are disclosed in Putney, S.D., et al. (Proc. Natl. Acad. Sci. (U.S.A.) 78:7350-7354 (1981)) and Gupta, A.P., et al. Nucl, Acids. Res., 12:5897-5911 (1984)).

In addition to being resistant to such exonucleases, nucleic acid molecules that contain phosphorothioate derivatives at restriction endonuclease cleavage recognition sites are resistant to such cleavage. Taylor, J.W., el al. (Nucl. Acids Res., 13:8749-8764 (1985)) discusses the endonuclease resistant properties of phosphorothioate nucleotide containing nucleic acid molecules.

The nuclease resistance of phosphorothioate bonds has been utilized in a DNA amplification protocol (Walker, T.G. et al. (Proc. Natl. Acad. Sci. (U.S.A.) 89:392-396 (1992)). In the Walker et al. method, phosphorothioate nucleotide derivatives are installed within a restriction endonuclease recognition site in one strand of a double-stranded DNA molecule. The presence of the phosphorothioate nucleotide derivatives protects that strand from cleavage, and thus results in the nicking of the unprotected strand by the restriction endonuclease. Amplification is accomplished by cycling the nicking and polymerization of the strands.

Similarly, this resistance to nuclease attack has been used as the basis for a modified "Sanger" sequencing method (Labeit, S. et al. (DNA 5:173-177 (1986)). In the Labeit et al. method, $^{35}$S-labeled phosphorothioate nucleotide derivatives were employed in lieu of the dideoxy nucleotides of the "Sanger" method.

In the most preferred embodiment, the phosphorothioate derivative is included in the primer. The nucleotide derivative may be incorporated into any position of the primer, but will preferably be incorporated at the 5'-terminus of the primer, most preferably adjacent to one another. Preferably, the primer molecules will be approximately 25 nucleotides in length, and contain from about 4% to about 100%, and more preferably from about 4% to about 40%, and most preferably about 16%, phosphorothioate residues (as compared to total residues). The nucleotides may be incorporated into any position of the primer, and may be adjacent to one another, or interspersed across all or part of the primer.

In one embodiment, the present invention can be used in concert with an amplification protocol, for example, PCR. In this embodiment, it is preferred to limit the number of phosphorothioate bonds of the primers to about 10 (or approximately half of the length of the primers), so that the primers can be used in a PCR reaction without any changes to the PCR protocol that has been established for non-modified primers. When the primers contain more phosphorothioate bonds, the PCR conditions may require adjustment, especially of the annealing temperature, in order to optimize the reaction.

The incorporation of such nucleotide derivatives into DNA or RNA can be accomplished enzymatically, using a DNA polymerase (Vosberg, H.P. et al., Biochemistry 16: 3533-3640 (1977); Burgers, P.M.J. et al., J. Biol. Chem. 254: 6889-6893 (1979); Kunkel, T.A., In: Nucleic Acids and Molecular Biology, Vol. 2, 124-135 (Eckstein, F. et al., eds.), Springer-Verlag, Berlin, (1988); Olsen, D.B. et al., Proc. Natl. Acad. Sci. (U.S.A.) 87:1451-1455 (1990); Griep, M.A. et al., Biochemistry 29:9006-9014 (1990); Sayers, J.R. et al., Nucl. Acids Res. 16:791-802 (1988)). Alternatively, phosphorothioate nucleotide derivatives can be incorporated synthetically into an oligonucleotide (Zon, G. et al., Anti-Canc. Drug Des. 6:539-568 (1991)).

The primer molecules are permitted to hybridize to a complementary target nucleic acid molecule, and are then extended, preferably via a polymerase, to form an extension product. The presence of the phosphorothioate nucleotides in the primers renders the extension product resistant to nuclease attack. As indicated, the amplification products containing phosphorothioate or other suitable nucleotide derivatives are substantially resistant to "elimination" (i.e. degradation) by "5'→3'" exonucleases such as T7 exonuclease or exonuclease, and thus a 5'→ 3' exonuclease will be substantially incapable of further degrading a nucleic acid molecule once it has encountered a phosphorothioate residue.

Since the target molecule lacks nuclease resistant residues, the incubation of the extension product and its template - the target - in the presence of a 5'→3' exonuclease results in the destruction of the template strand, and thereby achieves the preferential production of the desired single strand.

**D. Solid Phase Attachment of DNA**

The preferred method of determining the identity of the polymorphic site of a polymorphism involves nucleic acid hybridization. Although such hybridization can be performed in solution (Berk, A.J., et al. Cell 12:721-732 (1977); Hood, L.E., et al., In: Molecular Biology of Eukaryotic Cells: A Problems Approach, Menlo Park, CA: Benjamin-Cummings, (1975); Wetmer, J.G., Hybridization and Renaturation Kinetics of Nucleic Acids. Ann. Rev. Biophys, Bioeng. 5: 337-361 (1976); Itakura, K., et al., Ann. Rev. Biochem. 53:323-356, (1984)), it is preferable to employ a solid-phase hybridization assay (see, Saiki, R.K. et al., Proc. Natl. Acad. Sci. (U.S.A.) 86:6230-6234 (1989); Gilham et al., J. Amer. Chem. Soc. 86:4982 (1964) and Kremsky et al., Nucl. Acids Res. 15:3131-3139 (1987)).

Any of a variety of methods can be used to immobilize oligonucleotides to the solid support. One of the most widely used methods to achieve such an immobilization of oligonucleotide primers for subsequent use in hybridization-based assays consists of the non-covalent coating of these solid phases with streptavidin or avidin and the subsequent immobilization of biotinylaxed oligonucleotides (Holmstrom, K. et al., Anal. Biochem. 209:278-283 (1993)). Another known method (Running. J.A. et al., BioTechniques 8:276-277 (1990); Newton, C.R. et al. Nucl. Acids Res. 21 : 1155-1162 (1993)) requires the pre-coating of the polystyrene or glass solid phases with poly-L-Lys or poly L-Lys, Phe, followed by the covalent attachment of either amino- or sulfhydryl-modified oligonucleotides using bifunctional crosslinking reagents. Both methods have the disadvantage of requiring the use of modified oligonucleotides as well as a pre-treatment of the solid phase.

In another published method (Kawai, S et al., Anal. Biochem. 209:63-69 (1993)), short oligonucleotide probes were ligated together to form mummers and these were ligated into a phagemid vector. Following in vitro amplification and isolation of the single-stranded form of these phagemids, they were immobilized onto polystyrene plates and fixed by UV irradiation at 254 nm. The probes immobilized in this way were then used to capture and detect a biotinylated PCR product.

A method for the direct covalent attachment of short, 5'-phosphorylated primers to chemically modified polystyrene plates ("Covalink" plates, Nunc) has also been published (Rasmussen, S.R. et al., Anal. Biochem. 198:138-142 (1991)). The covalent bond between the modified oligonucleotide and the solid phase surface is introduced by condensation with a watersoluble carbodiimide. This method is claimed to assure a predominantly 5'-attachment of the oligonucleotides via their 5'-phosphates; however, it requires the use of specially prepared, expensive plates.

Most preferably, such immobilization of oligonucleotides (preferably between 15 and 30 bases) is accomplished using a method that can be used directly, without the need for any pre-treatment of commercially available polystyrene microwell plates (ELISA plates) or microscope glass slides. Since 96 well polystyrene plates are widely used in ELISA tests, there has been significant interest in the development of methods for the immobilization of short oligonucleotide primers to the wells of these plates for subsequent hybridization assays. Also of interest is a method for the immobilization to microscope glass slides, since the latter are used in the so-called Slide Immunoenzymatic Assay (SIA) (de Macario, E.C. et al., BioTechniques 3:138-145 (1985)).

The solid support can be glass, plastic, paper, etc. The support can be fashioned as a bead, dipstick, test tube, etc. In a preferred embodiment, the support will be a microtiter dish, having a multiplicity of wells. The conventional 96-well microtiter dishes used in diagnostic laboratories and in tissue culture are a preferred support. The use of such a support allows the simultaneous determination of a large number of samples and controls, and thus facilitates the analysis. Automated delivery systems can be used to provide reagents to such microtiter dishes. Similarly, spectrophotometric methods can be used to analyze the polymorphic sites, and such analysis can be conducted using automated spectrophotometers.

One aspect of the present invention concerns a method for immobilizing oligonucleotides for such analysis. In accordance with the method, any of a number of commercially available polystyrene plates can be used directly for the immobilization, provided that they have a hydrophilic surface. Examples of suitable plates include the Immulon (Registered Trade Mark) 4 plates (Dynatech) and the Maxisorp (Registered Trade Mark) plates (Nunc). The immobilization of the oligonucleotides to the plates is achieved simply by incubation in the presence of a suitable salt. No immobilization takes place in the absence of a salt, i.e., when the oligonucleotide is present in a water solution. Examples for suitable salts are: 50-250 mM NaCl; 30-100 mM 1-ethyl-3-(3'-dimethylaminopropyl)carbodiimide hydrochloride (EDC), pH 6.8; 50-150 mM octyldimethylamine hydrochloride, pH 7.0; 50-250 mM tetramethylammonium chloride. The immobilization is achieved by incubation, preferably at room temperature for 3 to 24 hours. After such incubation, the plates are washed, preferably with a solution of 10 mM Tris HCl, pH 7.5, containing 150 mM NaCl and 0.05% vol. Tween-20 (TNTw). The latter ingredient serves the important role of blocking all free oligonucleotide binding sites still present on the polystyrene surface, so that no nonspecific binding of oligonucleotides can take place during the subsequent hybridization steps. Using radioactively labeled oligonucleotides, the amount of immobilized oligonucleotioes per well was determined to be at least 500 fmoles. The oligonucleotides are immobilized to the surface of the plate with sufficient stability and can only be removed by prolonged incubations with 0.5 M NaOH solutions at elevated temperatures. No oligonucleotide is removed by washing the plate with water, TNTw (Tween 20), PBS, 1.5 M NaCl, or other similar solutions.

The immobilized oligonucleotides can be used to capture specific DNA sequences by hybridization. The hybridization is usually carried out in a solution containing 1.5 M NaCl and 10 mM EDTA, for 15 to 30 minutes at room temperature. Other hybridization conditions can also be used. More than 400 fmoles of a specific DNA sequence was found to hybridize to the immobilized oligonucleotide in one well. This DNA is bound to the initially immobilized oligonucleotide only via Watson-Crick hydrogen bonds can be easily removed from the wells by a brief wash with a 0.1 M NaOH solution, without removing the initially attached oligonucleotide from the plate. If the captured DNA fragment is nonradioactively labeled, e.g., with a biotin residue, the detection can be carried out using a suitable enzyme-linked assay.

Although no modifications have to be introduced into the synthetic oligonucleotides, the method also allows for the immobilization of labeled (e.g., biotinylated) oligonucleotides, if desired. The amount of oligonucleotide that can be immobilized in a single well of an ELISA plate by this method is at least 500 fmoles. The oligonucleotides thus immobilized onto the solid phase can hybridize to suitable templates and also participate in enzymatic reactions like template-directed extensions and ligations.

For high volume testing applications, it is desirable to use non-radioactive detection methods. Thus, the use of haptenated dideoxynucleotides is preferred; the use of biotinylated dideoxynucleotides is particularly preferred as such modification would render the incorporated base detectable by the standard avidin (or streptavidin) enzyme conjugates used in ELISA assays. The biotinylated ddNTPs are preferably prepared by reacting the four respective (3-aminopropyn-1-yl)nucleoside triphosphates with sulfosuccinimidyl 6-(biotinamido)hexanoate. Thus, (3-aminopropyn-1-yl) nucleoside 5'-triphosphates are prepared as described by Hobbs, F. W. (J. Org. Chem. 54:3420-3422 (1989)) and by Hobbs, F.W. et al. (U.S. Patent No. 5,047,519). The (3-aminopropyn-1-yl)nucleoside 5'-triphosphate (50 mol) is dissolved in 1 ml of pH 7.6, 1 M aqueous triethylammonium bicarbonate (TEAS). Sulfosuccinimidyl 6-(biotinamido) hexanoate sodium salt (Pierce, 55.7 mg, 100 mol is added and the solution is heated to 50°C in a stoppered tube for 2 hr. The reaction mixture is diluted to 10 ml with water and applied to a DEAE-Sephadex A-25-120 column. (1.6 x 19 cm). The column is eluted with a linear gradient of pH 7.6 aqueous TEAB (0.1 M to 1.0 M) and the eluent monitored at 270 nm. The late-eluting major peak is collected, stripped, and co-evaporated with ethanol. The crude product, containing biotinylated nucleoside triphosphate and, in some cases, contaminating starting material, is further purified by reverse phase column chromatography (Baker C-18 packing, 2 x 12 cm bed). The material is loaded in 0.1 M pH 7.6 TEAB and eluted with a step gradient of acetonitrile in 0.1 M pH 7.6 TEAB (O% to 36%, 2% increments, 8 ml/step). In all cases, the biotinylated product is more strongly retained and cleanly resolved from the starting material. Product-containing fractions are pooled, stripped, and co-evaporated with ethanol. The product is taken up in water and the yield calculated using the absorption coefficient for the starting nucleotide. The $^{3}$H NMR and $^{31}$P NMR spectra are consistent with the expected structure and confirm the absence of phosphorus containing or nucleotide-derived impurities. The materials are observed to be >99% pure by HPLC (Waters Bondapak C-18, 4.6 x 250 mm, 1 ml/min, 1 to 35% $CH_3CN$/pH 7/0.01 M triethylammonium acetate).

The synthesis of 5-(3-(6-biotinamido(hexanoamido) propyn-1-yl)-2',3'-dideoxyuridine-5'-triphosphate has an approximate yield of 25% (assuming = 12,400 at 291.5 nm); HPLC $t_x$ = 16.1 min.

The synthesis of 5-(3-(6-biotinamido(hexanoamido) propyn-1-yl)-2',3'-dideoxycytidine-5'-triphosphate has an approximate yield of 63% (assuming = 9,230 at 294.5 nm); HPLC $t_x$ = 19.4 min.

The synthesis of 7-(3-(6-biotinamido(hexanoamido) propyn-1-yl)-7-deaza-2',3'-dideoxyadenosine-5'-triphosphate has an approximate yield of 39% (assuming = 13.600 at 278.5 nm); HPLC $t_x$ = 23.1 min.

The synthesis of 7-(3-(6-biotinamido(hexanoamido) propyn-1-yl)-7-deaza-2',3'-dideoxyguanosine-5'-triphosphate has an approximate yield of 44% (assuming = 9,300 at 291 nm); HPLC $t_x$ = 21.2 min.

**E. Solid Phase Analysis of Polymorphic Sites**

**1. Polymerase-Mediated Analysis**

Although the identity of the nucleotide(s) of the polymorphic sites of the present invention can be determined in a variety of ways, an especially preferred method exploits the oligonucleotide-based diagnostic assay of nucleic acid sequence variation disclosed by Goelet, P. et al. (PCT Application WO92/15712). In this assay, a purified oligonucleotide having a defined sequence (complementary to an immediate proximal or distal sequence of a polymorphism) is bound to a solid support, especially a microtiter dish. A sample, suspected to contain the target molecule, or an amplification product thereof, is placed in contact with the support, and any target molecules present are permitted to hybridize to the bound oligonucleotide.

In one preferred embodiment, an oligonucleotide having a sequence that is complementary to an immediately distal sequence of a polymorphism is prepared using the above-described methods (and preferably that of Nikiforov, T. (U.S. Patent Application Serial No. 08/005,061, corresponding to WO94/16090, published 21 July 1994). The terminus of the oligionucleotide is attached to the solid support, as described, for example by Goelet, P. et al. (PCT Application WO92/15712), such that the 3'-end of the oligionucleotide can serve as a substrate for primer extension.

The immobilized primer is then incubated in the presence of a DNA molecule (preferably a genomic DNA molecule) having a single nucleotide polymorphism whose immediately 3'-distal sequence is complementary to that of the immobilized primer. Preferably, such incubation occurs in the complete absence of any dNTP (i.e. dATP, dCTP, dGTP, or dTTP), but only in the presence of one or more chain terminating nucleotide triphosphate derivatives (such as a dideoxy derivative), and under conditions sufficient to permit the incorporation of such a derivative on to the 3'-terminus of the primer. As will be appreciated, where the polymorphic site is such that only two or three alleles exist (such that only two or three species of dNTPs, respectively, could be incorporated into the primer extension product), the presence of unusable nucleotide triphosphate(s) in the reaction is immaterial. in consequence of the incubation, and the use of only chain terminating nucleotide derivatives, a single dideoxynucleotide is added to the 3'-terminus of the primer. The identity of that added nucleotide is determined by, and is complementary to, the nucleotide of the polymorphic site of the polymorphism.

in this embodiment, the nucleotide of the polymorphic site is thus determined by assaying which of the set of labeled nucleotides has been incorporated onto the 3'-terminus of the bound oligonucleotide by a primer-dependent polymerase. Most preferably, where multiple dideoxynucleotide derivatives are simultaneously employed, different labels will be used to permit the differential determination of the identity of the incorporated dideoxynucleotide derivative.

**2. Polymerase/Ligase-Mediated Analysis**

in an alternative embodiment, the identity of the nucleotide of the polymorphic site is determined using. a polymerase/ligase-mediated process. As in the above embodiment, an oligonucleotide primer is employed, that is complementary to the immediately 3'-distal invariant sequence of the SNP. A second oligonucleotide, is tethered to the solid phase via its 3'-end. The sequence of this oligonucleotide is complementary to the 5'-proximal sequence of the polymorphism being analyzed, but is incapable of hybridizing to the oligonucleotide primer.

These oligonucleotides are incubated in the presence of DNA containing the single nucleotide polymorphism that is to be analyzed, and at least one 2'. 5-deoxynucleotide triphosphate. The incubation reaction further includes a DNA polymerase and a DNA ligase. Thus, for example, where the polymorphism of clone 177-2 (Table 1) is being evaluated, and the tethered oligonucleotide could comprise the 3'-distal sequence of SEQ ID NO:2, the second oligonucleotide would have the 5'-proximal sequence of SEQ ID NO:1.

The tethered and soluble oligonucleotides are thus capable of hybridizing to the same strand of the single nucleotide polymorphism under analysis. The sequence considerations cause the two oligonucleotides to hybridize to the proximal and distal sequences of the SNP that flank the polymorphic site (X) of the polymorphism; the hybridized oligonucleotides are thus separated by a "gap" of a single nucleotide at the precise position of the polymorphic site.

The presence of a polymerase and a 2', 5'-deoxynucleotide triphosphate complementary to (X) permits ligation of the primer extended with the complementary 2', 5'-deoxynucleotide triphosphate to the immobilized oligo complementary to the distal sequence, a 2', 5'-deoxynucleotide triphosphate that is complementary to the nucleotide of the polymorphic site permits the creation of a ligatable substrate. The ligation reaction immobilizes the 2', 5'-deoxynucleotide and the previously soluble primer oligonucleotide to the solid support.

The identity of the polymorphic site that was opposite the "gap" can then be determined by any of several means. In a preferred embodiment, the 2', 5'-deoxynucleotide triphosphate of the reaction is labeled, and its detection thus reveals the identity of the complementary nucleotide of the polymorphic site. Several different 2', 5'-deoxynucleotide triphosphates may be present, each differentially labeled. Alternatively, separate reactions can be conducted, each with a different 2', 5'-deoxynucleotide triphosphate. In an alternative sub-embodiment, the 2', 5'-deoxynucleotide triphosphates are unlabeled, and the second, soluble oligonucleotide is labeled. Separate reactions are conducted, each using a different unlabeled 2', 5'-deoxynucleotide triphosphate. The reaction that contains the complementary nucleotide permits the ligatable substrate to form, and is detected by detecting the immobilization of the previously soluble oligonucleotide.

**F. Signal-Amplification**

The sensitivity of nucleic acid hybridization detection assays may be increased by altering the manner in which detection is reported or signaled to the observer. Thus, for example, assay sensitivity can be increased through the use of detectably labeled reagents. A wide variety of such signal amplification methods have been designed for this purpose. Kourilsky et al. (U.S. Patent 4,581,333) describe the use of enzyme labels to increase sensitivity in a detection assay. Fluorescent labels (Albarella et al., EP 144914), chemical labels (Sheldon III et al., U.S. Patent 4,582.789; Albarelta et al., U.S. Patent 4,563,417), modified bases (Miyoshi et al., EP 119448), etc. have also been used in an effort to improve the efficiency with which hybridization can be observed.

It is preferable to employ fluorescent, and more preferably chromogenic (especially enzyme) labels, such that the identity of the incorporated nucleotide can be determined in an automated, or semi-automated manner using a spectrophotometer.

**IV. The Use of SNP Genotyping in Methods of Genetic Analysis**

**A. General Considerations for Using Single Nucleotide Polymorphisms in Genetic Analysis**

The utility of the polymorphic sites of the present invention stems from the ability to use such sites to predict the statistical probability that two individuals will have the same alleles for any given polymorphisms.

Statistical analyses of SNPs can be used for any of a variety of purposes. Where a particular animal has been previously tested, such testing can be used as a "fingerprint" with which to determine if a certain animal is, or is not that particular animal.

Where a putative parent or both parents of an individual have been tested, the methods of the present invention may be used to determine the likelihood that a particular animal is or is not the progeny of such parent or parents. Thus, the detection and analysis of SNVs can be used to exclude paternity of a male for a particular individual (such as a stallion's paternity of a particular foal), or to assess the probability that a particular individual is the progeny of a selected female (such as a particular foal and a selected mare).

As indicated below, the present invention permits the construction of a genetic map of a target species. Thus, the particular array of polymorphisms identified by the methods of the present invention can be correlated with a particular trait, in order to predict the predisposition of a particular animal (or plant) to such genetic disease, condition, or trait. As used herein, the term "trait" is intended to encompass "genetic disease," "condition," or "characteristics." The term, "genetic disease" denotes a pathological state caused by a mutation, regardless of whether that state can be detected or is asymptomatic. A "condition" denotes a predisposition to a characteristic (such as asthma, weak bones, blindness, ulcers, cancers, heart or cardiovascular illnesses, skeleto-muscular defects, etc.). A "characteristic" is an attribute that imparts economic value to a plant or animal. Examples of characteristics include longevity, speed, endurance, rate of aging, fertility, etc.

**B. Identification and Parentage Verification**

The most useful measurements for determining the power of an identification and paternity testing system are: (i) the "probability of identity" (p(ID)) and (ii) the "probability of exclusion" (p(exc)). The p(ID) calculates the likelihood that two random individuals will have the same genotype with respect to a given polymorphic marker. The p(exc) calculates the likelihood, with respect to a given polymorphic marker, that a random male will have a genotype incompatible with him being the father in an average paternity case in which the identity of the mother is not in question. Since single genetic loci, including loci with numerous alleles such as the major histocompatibility region, rarely provide tests with adequate statistical confidence for paternity testing, a desirable test will preferably measure multiple unlinked loci in parallel. Cumulative probabilities of identity or non-identity, and cumulative probabities of paternity exclusion are determined for these multi-locus tests by multiplying the probabilities provided by each locus.

The statistical measurements of greatest interest are: (i) the cumulative probability of non-identity (cum *p* (nonID)), and (ii) the cumulative probability of paternity exclusion (cum *p*(exc)).

The formulas used for calculating these probability values are given below. For simplicity these are given first for 2-allele loci, where one allele is termed type A and the other type B. In such a model, four genotypes are possible: AA, AB, BA, and BB (types AB and BA being indistinguishable biochemically). The allelic frequency is given by the number of times A (f(A), the frequency of A is denoted by "p") or B (f(B), the frequency of B is denoted by "q," where q = 1-p) is found in the haploid genome. The probability of a given genotype at a given locus:

$$Homozygote: p(AA)= p^2$$

$$Single\ Heterozygote: p(AB)= p(BA)= pq =p(1\text{-}p)$$

$$Both\ Heterozygotes: p(AB+BA)= 2pq = 2p(1\text{-}p)$$

$$Homozygote: p(BB)= q^2 = (1\text{-}p)^2$$

The probability of identity at one locus (i.e the probability that two individuals, picked at random from a population will have identical genotypes at a given locus) is given by the equation:

$$p(ID) = (p^2)^2 + (2pq)^2 + (q^2)^2$$

The cumulative probability of identity for n loci is therefore given by the equation:

$$cum\ p(ID) = \subseteq p(ID_1)p(ID_2)p(ID_3)....p(ID_n)$$

The cumulative probability of non-identity for n loci (i.e. the probability that two individuals will be different at 1 or more loci) is given by the equation:

$$cum\ p(nonID) = 1 - cum\ p(ID)$$

The probability of parentage exclusion (representing the probability that a random male will have a genotype, with respect to a given locus, that makes him incompatible as the sire in an average paternity case where the identity of the mother is not in question) is given by the equation:

$$p(exc) = pq(1\text{-}pq)$$

The probability of non-exclusion (representing the probability at a given locus that a random male will not be biochemically excluded as the sire in an average paternity case) is given by the equation:

$$p(non\text{-}exc) = 1 - p(exc)$$

The cumulative probability of non-exclusion (representing the value obtained when n loci are used) is thus:

$$cum\ p(non\text{-}exc) = \subseteq p(non\text{-}exc_1)p(non\text{-}exc_2)p(non\text{-}exc_3)....p(non\text{-}exc_n)$$

The cumulative probability of exclusion (representing the probability, using a panel of n loci, that a random male will be biochemically excluded as the sire in an average paternity case where the mother is not in question) is given by the equation:

$$cum\ p(exc) = 1 - cum\ p(non\text{-}exc)$$

These calculations may be extended for any number of alleles at a given locus. For example, the probability of identity $p$(ID) for a 3-allele system where the alleles have the frequencies in the population of p, q and r, respectively, is equal to the sum of the squares of the genotype frequencies:

$$p(ID) = p^4 + (2pq)^2 + (2qr)^2 + (2pr)^2 + r^4 + q^4$$

Similarly, the probability of exclusion for a three allele system is given by:

$$p(exc) = pq(1-pq) + qr(1-qr) + pr(1-pr) + 3pqr(1-pqr)$$

In a locus of n alleles, the appropriate binomial expansion is used to calculate $p$(ID) and $p$(exc).

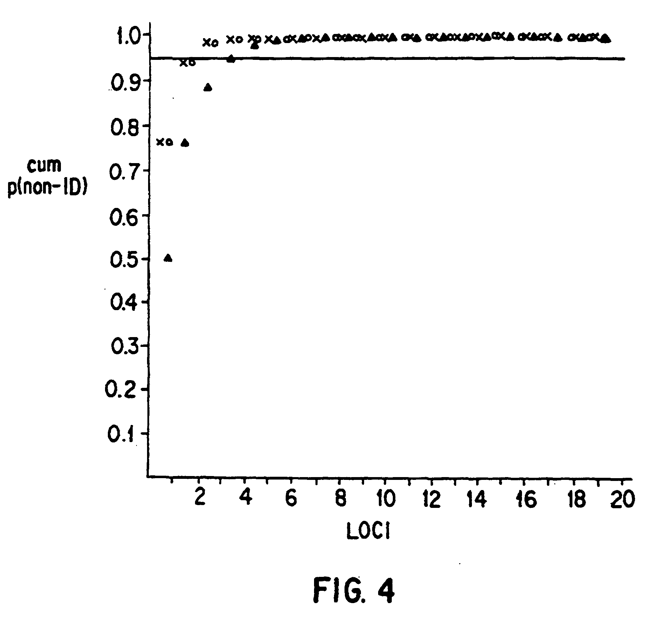

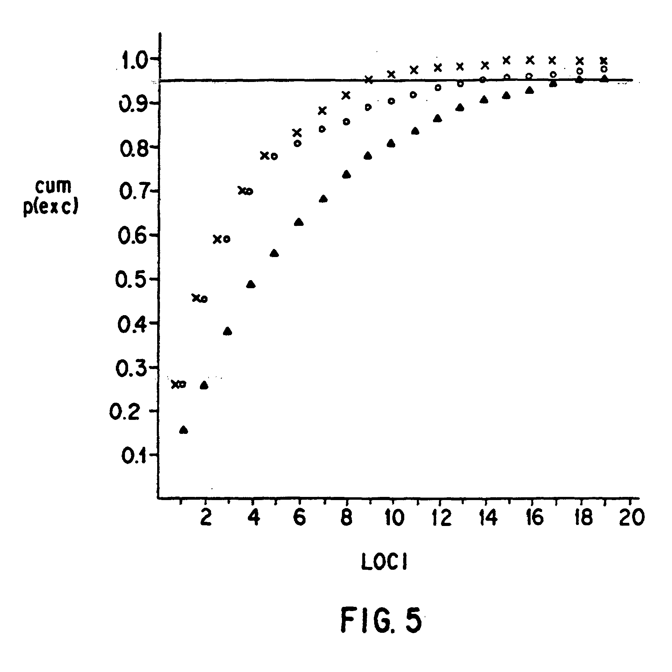

Figures 4 and 5 show how the cum $p$(nonID) and the cum $p$(exc) increase with both the number and type of genetic loci used. It can be seen that greater discriminatory power is achieved with fewer markers when using three allele systems.

In Figures 4 and 5, the triangles trace the increase in probability values with increasing numbers of loci with two alleles where the common allele is present at a frequency of p = 0.79. The crosses in Figures 4 and 5 show the same analysis for increasing numbers of three-allele loci where p = 0.51, q = 0.34 and r = 0.15.

The choice between whether to use loci with 2, 3 or more alleles is however largely influenced by the above-described biochemical considerations. A polymorphic analysis test may be designed to score for any number of alleles at a given locus. If allelic scoring is to be performed using gel electrophoresis, each allele should be easily resolvable by gel electrophoresis. Since the length variations in multiple allelic families are often small, human DNA tests using multiple allelic families include statistical corrections for mistaken identification of alleles. Furthermore, although the appearance of a rare allele from a multiple allelic system may be highly informative, the rarity of these alleles makes accurate measurements of their frequency in the population extremely difficult. To correct for errors in these frequency estimates when using rare alleles, the statistical analysis of this data must include a measure of the cumulative effects of uncertainty in these frequency estimates. The use of these multiple allelic systems also increases the likelihood that new or rare alleles in the population will be discovered during the course of large population screening. The integrity of previously collected genetic data would be empirically revised to reflect the discovery of a new allele.

In view of these considerations, although the use of loci with many alleles could potentially offer some short-term advantages (because fewer loci would need to be screened), it is preferable to perform polymorphic analyses using loci with fewer alleles that are: (i) more frequently represented, and (ii) easier to measure unambiguously. Tests of this type can achieve the same power of discrimination as tests based on more highly polymorphic loci, provided the same total number of alleles is collected from a series of unlinked loci.

## C. Gene Mapping and Genetic Trait Analysis Using SNPs

The polymorphisms detected in a set of individuals of the same species (such as humans, horses, etc.), or of closely related species, can be analyzed to determine whether the presence or absence of a particular polymorphism correlates with a particular trait.

To perform such polymorphic analysis, the presence or absence of a set of polymorphisms (i.e. a "polymorphic array") is determined for a set of the individuals, some of which exhibit a particular trait, and some of which exhibit a mutually exclusive, characteristic (for example, with respect to horses, brittle bones vs. non-brittle bones; maturity onset blindness vs. no blindness; predisposition to asthma, cardiovascular disease vs. no such predisposition). The alleles of each polymorphism of the set are then reviewed to determine whether the presence or absence of a particular allele is associated with the particular trait of interest. Any such correlation defines a genetic map of the individual's species. Alleles that do not segregate randomly with respect to a trait can be used to predict the probability that a particular animal will express that characteristic. For example, if a particular polymorphic allele is present in only 20% of the members of a species that exhibit a cardiovascular condition, then a particular member of that species containing that allele would have a 20% probability of exhibiting such a cardiovascular condition. As indicated, the predictive power of the analysis is increased by the extent of linkage between a particular polymorphic allele and a particular characteristic. Similarly, the predictive power of the analysis can be increased by simultaneously analyzing the alleles of multiple polymorphic loci and a particular trait. In the above example, if a second polymorphic allele was found to also be present in 20% of members exhibiting the cardiovascular condition, however, all of the evaluated members that exhibited such a cardiovascular condition had a particular combination of alleles for these first and second polymorphisms, then a particular member containing both such alleles would have a very high probability of exhibiting the cardiovascular condition.

The detection of multiple polymorphic sites permits one to define the frequency with which such sites independently segregate in a population. If, for example, two polymorphic sites segregate randomly, then they are either on separate chromosomes, or are distant to one another on the same chromosome. Conversely, two polymorphic sites that are co-inherited at significant frequency are linked to one another on the same chromosome. An analysis of the frequency of segregation thus permits the establishment of a genetic map of markers. Thus, the present invention provides a means for mapping the genomes of plants and animals.

The resolution of a genetic map is proportional to the number of markers that it contains. Since the methods of the present invention can be used to isolate a large number of polymorphic sites, they can be used to create a map having any desired degree of resolution.

The sequencing of the polymorphic sites greatly increases their utility in gene mapping. Such sequences can be used to design oligonucleotide primers and probes that can be employed to "walk" down the chromosome and thereby identify new marker sites (Bender, W. et al., J. Supra. Molec. Struc. 10(suppl.):32 (1979); Chinault, A.C. et al., Gene 5:111-126 (1979); Clarke, L. et al., Nature 287:504-509 (1980)).

The resolution of the map can be further increased by combining polymorphic analyses with data on the phenotype of other attributes of the plant or animal whose genome is being mapped. Thus, if a particular polymorphism segregates with brown hair color, then that polymorphism maps to a locus near the gene or genes that are responsible for hair color. Similarly, biochemical data can be used to increase the resolution of the genetic map. In this embodiment, a biochemical determination (such as a serotype, isoform, etc.) is studied in order to determine whether it co-segregates with any polymorphic site. Such maps can be used to identify new gene sequences, to identify the causal mutations of disease, for example.

Indeed, the identification of the SNPs of the present invention permits one to use complimentary oligonucleotides as primers in PCR or other reactions to isolate and sequence novel gene sequences located on either side of the SNP. The invention includes such novel gene sequences. The genomic sequences that can be clonally isolated through the use of such primers can be transcribed into RNA, and expressed as protein. The present invention also includes such protein, as well as antibodies and other binding molecules capable of binding to such protein.

The invention is illustrated below with respect to two of its embodiments -- horses and humans. However, because the fundamental tenets of genetics apply irrespective of species, such illustration is equally applicable to any other species. Those of ordinary skill would therefore need only to directly employ the methods of the above invention to isolate SNPs in any other species, and to thereby conduct the genetic analysis of the present invention.

As indicated above, LOD scoring methodology has been developed to permit the use of RFLPs to both track the inheritance of genetic traits, and to construct a genetic map of a species (Lander, S. et al., Proc. Natl. Acad. Sci. (U.S.A.) 83:7353-7357 (1986); Lander, S. et al., Proc. Natl. Acad. Sci. (U.S.A.) 84:2363-2367 (1987); Donis-Keller, H. et al., Cell 51:319-337 (1987); Lander, S. et al., Genetics 121:185-199 (1989)). Such methods can be readily adapted to permit their use with the polymorphisms of the present invention. Indeed, such polymorphisms are superior to RFLPs and STRs in this regard. Due to the frequency of SNPs, it is possible to readily generate a dense genetic map. Moreover, as indicated above, the polymorphisms of the present invention are more stable than typical (VNTR-type) RFLP polymorphisms.

The polymorphisms of the present invention comprise direct genomic sequence information and can therefore be typed by a number of methods. In an RFLP or STR-dependent map, the analysis must be gel-based, and entail obtaining an electrophoretic profile of the DNA of the target animal. In contrast, an analysis of the polymorphisms (SNPs) of the present invention may be performed using spectrophotometric methods, and can readily be automated to facilitate the analysis of large numbers of target animals.

Having now generally described the invention, the same will be more readily understood through reference to the following examples of the isolation and analysis of equine polymorphisms which are provided by way of illustration, and are not intended to be limiting of the present invention.

## EXAMPLE 1

DISCOVERY OF EQUINE POLYMORPHISMS

As an initial step in the identification of equine polymorphisms, small shotgun libraries were prepared from genomic DNA isolated from peripheral blood leukocytes which had been purified on a Ficoll-hypaque density gradient from the blood of a single, 15 year old thoroughbred gelding (John Henry). This DNA was simultaneously digested to completion with Bam HI and Pst I and either used directly or after size fractionation on agarose gels.

Vector pLT14 (a variant of the Stratagene plasmid pKSM13(-)) was digested with Barn HI and Pst I and linearized DNA was purified from an agarose gel. For both vector and size-fractionated genomic DNA, agarose plugs were solubilized in saturated sodium iodide and the DNA was subsequently immobilized on glass powder. After washing, the DNA was eluted with water and ethanol precipitated with glycogen carrier.

Ligations with varying vector/insert ratios were effectuated with T4 DNA ligase at 4°C. E. coli strain XLI was transformed with ligation mixtures and plated on LB agar containing 100 g/ml ampicillin. Approximately 50,000 clones were generated in several different experiments using size fractionated or unfractionated insert DNA. Unplated transformed cells were stored at -70°C in 7% DMSO. Colonies were streaked for isolation and small scale plasmid preparations were performed to determine the size of inserted equine DNA. Larger scale preparations were performed with Qiagen chromatography.

The sequence of the first 200-300 nucleotides of the genomic insert was determined by the chain terminating dideoxynucleoside method with T7 DNA polymerase from primers complementary to plasmid sequences. This information was used to design synthetic oligonucleotide primers complementary to the equine sequence to be employed in PCR reactions.

In most cases, two sets of PCR primers (generally 25-mers) were synthesized. The first set was used to amplify, under a standardized set of conditions, from genomic DNA. The products of these reactions were diluted and used as template DNA in a second PCR using nested primers slightly internal to the original set. The products of these two reactions were compared to those obtained using the original plasmid DNA as template. In most cases, it was possible to obtain high quality, single-species products using this procedure with no attempt to optimize reaction conditions for any particular pair of primers.

Two different methods were used to screen amplified DNA from horses for polymorphic sequences. Initially, PCR fragments from a panel of 6 horses were digested with a panel of restriction endonucleases having 4 base recognition sites. The products of these reactions were analyzed by acrylamide gel electrophoresis on 5% - 7.5% nondenaturing gels. Digestion products which showed variability when hybridized to different members of the panel were subjected to DNA sequence analysis. Later, DNA sequencing was used directly to screen for polymorphic sites. The PCR fragments from five unrelated horses were electroeluted from acrylamide gels and sequenced using repetitive cycles of thermostable Taq polymerase reaction in the presence of a mixture of dNTPs and fluorescent ddNTPs. The products were then separated and analyzed using the automated DNA sequencing instrument of Applied Biosystems, Inc. The data was analyzed using ABI software. Differences between sequences of different animals were identified by the software and confirmed by inspecting the relevant portion of the chromatograms on the computer screen. Differences were concluded to be a DNA polymorphism only if the data was available for both strands, and/or present in more than one haploid example among the five horses tested.

## EXAMPLE 2

## CHARACTERIZATION OF EQUINE POLYMORPHISMS

The program of identification and characterization of polymorphic DNA sequences in randomly selected fragments was continued such that approximately 550 plasmids have been characterized to this level. The sequences adjacent to the cloning sites was determined for 200 of these plasmids. Inserts of these sequenced plasmids ranged in size from 0.25 to 3.5 kb. Using this sequence information, oligonucleotide primers were designed to enable PCR amplification of the same genomic region from different horses.

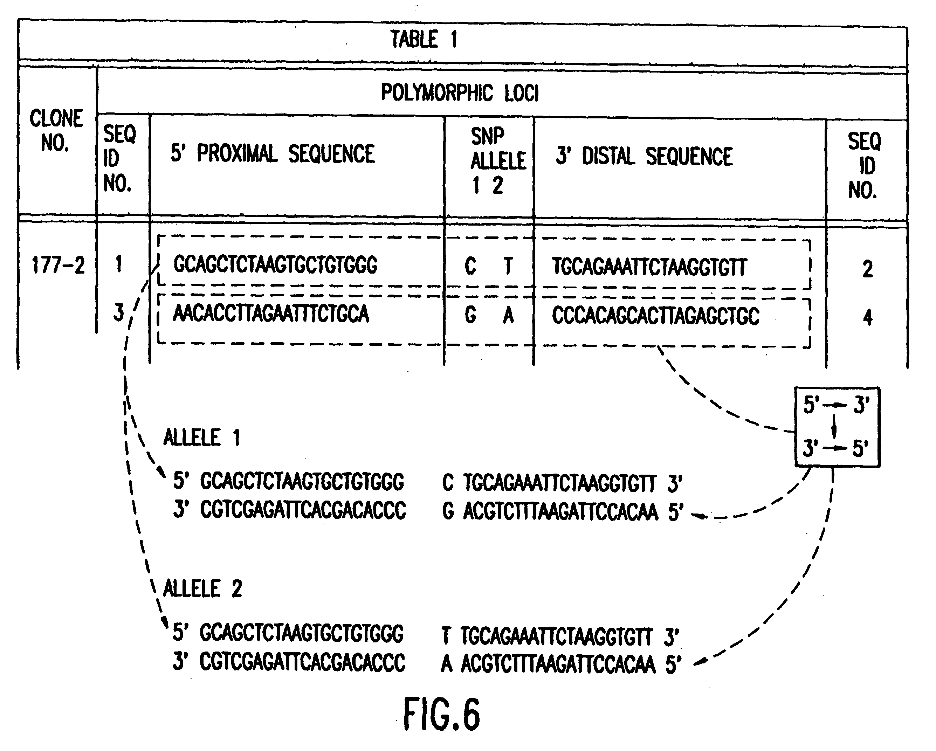

In order to identify the nucleotides present at polymorphic sites, PCR fragments from 5 horses were purified from acrylamide gels by electroelution and completely sequenced using Taq polymerase "Cycle" sequencing biochemistry and automated sequencing equipment. Results from the 5 horses were analyzed by computer and visually confirmed. DNA sequence variants discovered by this method were scored only if the sequence was obtained on both strands and the variant sequence had been found in more than one haploid example. The 18 clones of Table 1 comprise a subset of identified SNPs. In Table 1, the immediately 5'-proximal sequence, the identity of the nucleotide of the polymorphic site, and the immediately 3'-distal sequence of each SNP is presented. For each SNP, Such sequences are shown in the horizontal rows. The sequences of double-stranded DNA in Table 1 is presented in compliance with the Sequence Listing requirements of the United States Patent and Trademark Office. Thus, all sequences are presented in the same orientation (5'→3'). The organization of the Table is illustrated in Figure 6 with respect to an illustrative SNP, clone 177-2. This SNP has a polymorphic site capable of having either a C or a T in one strand, and a G or A in the opposite strand. The 5'-proximal DNA sequence that immediately precedes the polymorphic site in the C/T strand is designated as SEQ ID NO:1. The 3'-distal sequence that immediately follows the polymorphic site in the C/T strand is designated as SEQ ID NO:2. The 5'-proximal DNA sequence that immediately precedes the polymorphic site in the G/A strand is designated as SEQ ID NO:3. The 3'-distal sequence that immediately follows the polymorphic site in the G/A strand is designated as SEQ ID NO:4. Bearing in mind that the sequences are written in the same orientation (5'→3'), it will be seen that the sequences of SEQ ID NO:1 and SEQ ID NO:4 are complimentary; similarly, the sequences of SEQ ID NO:2 and SEQ ID NO:3 are complimentary. The sequences that flank a particular polymorphic site are thus obtained by combining the proximal sequence of one row with the distal sequence also shown in the same row.

TABLE 1

| CLONE | SEQ ID NO. | 5' PROXIMAL SEQUENCE | POLYMORPHIC LOCI — IDENTIFIED SNP ALLELE 1 | POLYMORPHIC LOCI — IDENTIFIED SNP ALLELE 2 | 3' DISTAL SEQUENCE | SEQ ID NO. |
|---|---|---|---|---|---|---|
| 177-2 | 1 | GCAGCTCTAAGTGCTGTGG | C | T | TGCAGAAATTCTAAGGTGTT | 2 |
| | 3 | AACACCTTAGAATTTCTGCA | G | A | CCCACAGCACTTAGAGCCTGC | 4 |
| 595-3 | 5 | AGCTCTGGGATGATCTACTA | A | G | TGAGGGAAAAATGATGATGC | 6 |
| | 7 | GCCATCATCATTTTTGTCCTCA | T | C | TAGTGGATCATCCCAGAGCT | 8 |
| 090-2 | 9 | AAAACTAATTTGATTGCCAT | G | A | AAAGTCAGAACAATGATTGC | 10 |
| | 11 | GCAATCATTGTTCTGACTTT | C | T | ATGGCCATCAAATTAGTTTT | 12 |
| 324-1 | 13 | CACAAGGCCCAAGAACAGGA | T | C | TGAGTTCAGCGAGTGTCAGA | 14 |
| | 15 | TCTGACACTCCCTGAACTCA | A | G | TCCTGTTCTTGGGCCTTGTG | 16 |
| 129-1 | 17 | TGGGAAAGACCCACATTATTT | T | A | GTTCCCTTTGTTGTTCAGACC | 18 |
| | 19 | GGTCTGAAACAAAAGGGAAC | A | T | AAATAATGTGGTCTTTCCCA | 20 |
| 007-1 | 21 | CATGAGTAAGAAGCATCCGG | G | C | CCATGGAGTCATAGATAAGT | 22 |
| | 23 | ACTTATCTATGACTCCATGG | C | G | CCGGATGCTTCTTACTCATG | 24 |
| 324-2 | 25 | CCCAAGAACAGGATTGAGTT | C | T | AGCGAGTGTCAGAGTTGTGT | 26 |
| | 27 | NCACACTCTGACACTGGCT | G | A | AAGGCCCCCATTTCTTGCT | 28 |
| 177-3 | 29 | AGCAAGAAATGGGGGGCCTT | A | C | GTCCTACAATTGCCAGGAAG | 30 |
| | 31 | CTTCCTGGCAATTTTAGGAC | T | C | ATATATATATATATGATATTC | 32 |
| 496-1 | 33 | GAATATCAATATATATATAT | G | C | TGTGTGTGTGTGTATTTGCT | 34 |
| | 35 | ACAATTACCACACACACACA | T | T | NTATATATATATTGATATTC | 36 |
| 007-3 | 37 | GCCATAATTAAGCCTGTATT | A | G | GTTTGTTTTAAATTTTGTGA | 38 |
| | 39 | TCACAAAATTTAAACAAAC | T | C | AATACAGGCTTAATTATGCC | 40 |
| 459-1 | 41 | GTGTAGAGTAGTTCAAGGAC | A | C | ATGTCTTATACCTCCCTTTT | 42 |
| | 43 | AAAAGGAGGTATAAGACAT | T | G | GTCCTTGAACTACTCTACAC | 44 |
| 085-1 | 45 | GTAACGGAGAGCAGGCCTT | C | G | CCTGCTGAAGCCTCAGACCG | 46 |
| | 47 | CCGTTCTGAGGCTTCAGCAGG | G | C | AAGGCCTGCTCTCCGTTCAC | 48 |
| 007-2 | 49 | CTGCTCTTTAGACTATGACC | G | A | TCAACCTTGCATCATGGAGCT | 50 |
| | 51 | AGCTCATGATGCAAGGTTGA | C | T | GGTCATAGTCTAAAGAGCAG | 52 |
| 474-1 | 53 | TTTGAGCTGGGACCTCAGTC | T | A | TCTCCTGCCTTTAGACTCGA | 54 |
| | 55 | TCGAGTCTAAGGCAGGAGA | A | T | GACTGAGGTCCCAGCTCAAA | 56 |
| 178-1 | 57 | GAACCTCTGGGCCGTGGATA | T | G | TTGTTCAGAAGCACACAGGTGA | 58 |
| | 59 | TCACCTGTGCTTCTGAACAA | T | C | TATCCACGGCCCAGAGGGTTC | 60 |
| 595-2 | 61 | GTATTTGCTTAGCTCTGGAT | A | C | ATCCACTAATGAGGGAAAAA | 62 |
| | 63 | TTTTTCCCTCATTAGTGGAT | T | C | ATCCCAGAGGCTAGCAAATAC | 64 |
| 177-1 | 65 | GAAGTTGTCGGACAGAGTGTG | C | A | AGAGATGCAGCTCTAAGTGC | 66 |
| | 67 | GCACTTAGAGCTTGCATTCT | G | T | CACATCTGTCCCACACTTC | 68 |
| 459-2 | 69 | CCATTAGTAAGCCTTTTACAA | C | G | GTCCCAAAAGTCTGGGATTC | 70 |
| | 71 | GAATCCCAGACTATTTATTAC | G | C | TTGTGGAGGCTTCCTCATGG | 72 |

The present specification refers to the above sequences by their sequence ID numbers (i.e. SEQ ID NO). To facilitate such disclosure, algebraic notation (such as "2n+1") is employed, in accordance with conventional algebra. Thus, the designation "SEQ ID NO:(2n+1)" denotes SEQ ID NO:5 where n=2, and SEQ ID NO:7 where n=3, etc.

## EXAMPLE 3

ALLELIC FREQUENCY ANALYSIS OF EQUINE POLYMORPHISMS IN SMALL POPULATION STUDIES

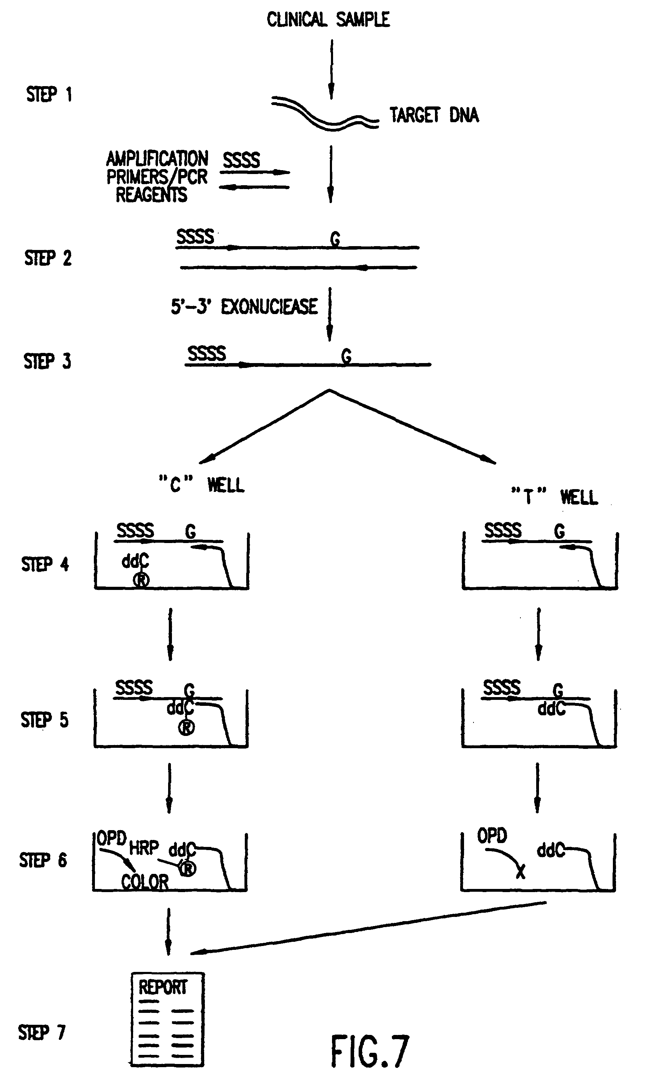

Small population studies (50 - 60 animals) of these DNA sequence polymorphisms has been carried out on a number of these polymorphic sites using Genetic Bit Analysis (GBA), the preferred solid-phase, single nucleotide interrogation system (Goelet, P. et al. (WO 92/15712). The 7 steps of the most preferred embodiment is illustrated in Figure 7:

Step 1: DNA preparation.
Step 2: Amplification of Target Sequence. After DNA is prepared from the sample, a specific region of the sample genome (locus) is amplified using the PCR. One of the PCR primers is modified with four phosphorothioate linkages at the 5'-end.
Step 3: Exonuclease Digestion and the Generation of Single-Stranded Template. The PCR product is digested with exonuclease, leaving the phosphorothioated strand intact.
Step 4: Hybridization to Capture the Amplified Template. The template strand is next hybridized to the appropriate GBA primer that is immobilized on the surface of a microtiter well.
Step 5: Single Base Extension with Polymerase. DNA polymerase and haptenated ddNTPs are used to extend the GBA primer by one base in a template-dependent manner.
Step 6: Colorimetric detection of the Extension Product. After the template is washed away using NaOH, the haptenated base is detected using an anti-hapten conjugate and the appropriate colorimetric substrate.
Step 6: Computer-Assisted interpretation of Genotype. The colorimetric data from a number of loci is converted to an SNP genotype for the particular individual tested.

The method is preferably conducted in the following manner:

**GBA Template Preparation.**

Amplification of genomic sequences was performed using the polymerase chain reaction (PCR). In a first step, one hundred nanograms of genomic DNA was used in a reaction mixture containing each first round primer at a concentration of 2 M and 10 mM Tris pH 8.3, 50 mM KCl, 1.5 mM $MgCl_2$, 0.01% gelatin; and 0.05 units per I Taq DNA Polymerase (AmpliTaq (Registered Trade Mark), Perkin Elmer).

To obtain single-stranded template for use with solid-phase immobilized primer, either of two methods may be used. First, the amplification may be mediated using primers that contain 4 posphorothioate-nucleotide derivatives, as taught by Nikiforov, T. (U.S. Patent Application Serial No. 08/005,061, corresponding to WO94/16090, published 21 July 1994). Alternatively, a second round of PCR may be performed using "asymmetric" primer concentrations. The products of the first reaction are diluted 1/1000 in a second reaction. One of the second round primers is used at the standard concentration of 2 M white the other is used at 0.08 M. Under these conditions, single stranded molecules are synthesized during the reaction.

**Solid phase immobilization of nucleic acids.**

For the GBA procedure, solid-phase attachment of the template-primer complex simplifies washes, buffer exchanges, etc.; and in principle this attachment can be either via the template or the primer. In practice, however, especially when non gel-based detection methods are employed, attachment via the primer is preferable. This format allows the use of stringent washes (e.g., 0.2 N NaOH) to remove impurities and reaction side products while retaining the haptenated dideoxynucleotide covalently linked to the 3'-end of the primer.

Therefore, for GBA reactions in 96-well plates (Nunc Nunclon (Registered Trade Mark), plates, Roskilde, Denmark), the GBA primer was covalently coupled to the plate. This was accomplished by incubating 10 pmoles of primer having a 5'-amino group per well in 50 of 3 mM sodium phosphate buffer, pH 6, 20 mM 1-ethyl-3-(3-dimethylaminopropyl)-carbodiimide (EDC) overnight at room temperature. After coupling, the plate was washed three times with TNTw.

**GBA in Microwell Plates.**

Hybridization of single-stranded DNA to primers covalently coupled to 96-well plates was accomplished by adding an equal volume of 3 M NaCl, 20 mM EDTA to the single-stranded PCR product and incubating each well with 20 I of this mixture at 20°C for 30 minutes. The plate was subsequently washed three times with TNTw. Twenty I of polymerase extension mix containing ddNTPs (3 M each, one of which was biotinylated, 5 mM DTT, 7.5 mM sodium isocitrate, 5 mM MnCl$_2$, 0.04 units per I of Klenow DNA polymerase and incubated for 5 minutes at room temperature.

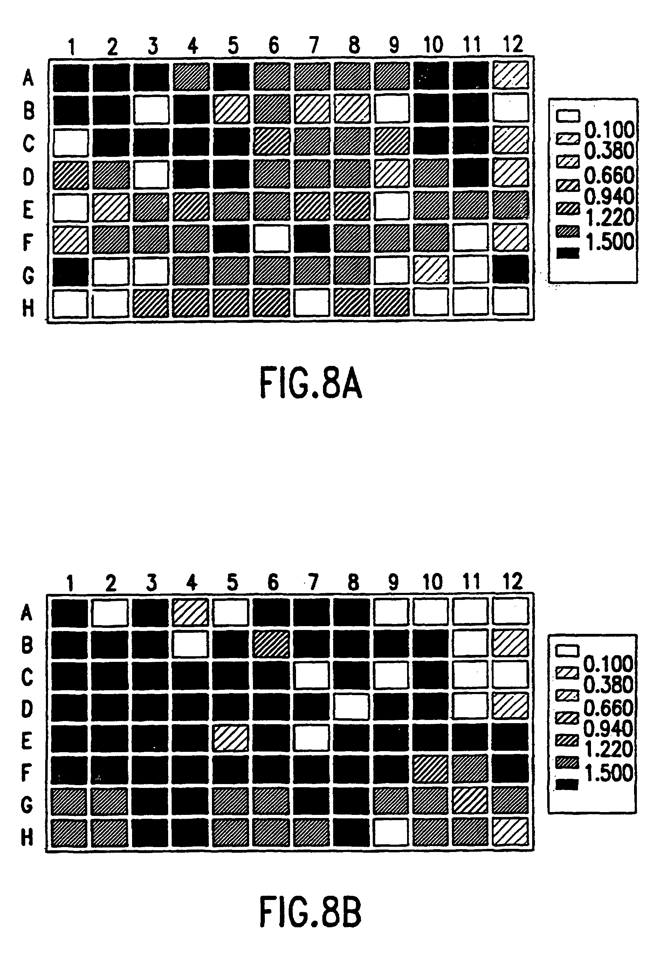

Following the extension reaction, the plate was washed once with TNTw. Template strands were removed by incubating wells with 50 µl of 0.2 N NaOH for 5 minutes at room temperature, then washing the well with another 50 µl of 0.2 N NaOH. The plate was then washed three times with TNTw. Incorporation of biotinylated ddNTPs was measured by an enzyme-linked assay. Each well was incubated with 20 µl of streptavidin-conjugated horseradish peroxidase (1/1000 dilution in TNTw of product purchased from BRL, Gaithersburg, MD) with agitation for 30 minutes at room temperature. After washing 5 times with TNTw, 100 µl of o-phenylenediamine (OPD, 1 mg/ml in 0.1 M citric acid, pH 4.5) (BRL) containing 0.012% H$_2$O$_2$ was added to each well. The amount of bound enzyme was determined kinetically with a Molecular Devices model "Vmax" 96-well spectrophotometer. Figures 8A and 8B illustrate how horse parentage data appears at the microtiter plate level. In standard horse parentage testing, samples are arrayed 85 to a plate (columns 1-11) plus controls (column 12). For each horse locus the presence of the two known alleles is determined by base specific interrogation on separate plates. The two plates shown in figures 8A and 8B are identical in PCR template and GBA primer and differ only in the biotinylated ddNTP that was used in the extension reaction (biotin-ddCTP in Figure 8A and biotin-ddTTP in Figure 8B). Upon addition of the colorimetric reagent (OPD), the absorbance of the resultant, color was measured in a Molecular Devices microtiter plate reader and the raw data generated in milliOD/min per well. The two raw data gray scale representations of the absorbance data for these plates are shown in the figures arranged in the exact same order as on the microtiter plates. Gray scale intensity correlates directly with color production. At this biallelic locus the bases detected are C (Figure 8A) and T (Figure 8B). Approximately 40% of horses tested to date are heterozygotes (the sample in well A1, for example) and the remaining homozygous for C (A2, for example) or T (B3, for example). Synthetic template controls include a control C homozygote (well E12), a control T homozygots (well F12) and a control heterozygote (well G12). Scale refers to milliOD/min at 450 nm. Most positive samples had signals above 100 in this case. In this format, for a 28 biallelic marker panel horse parentage test, 56 such plates would be required for complete typing of the 85 horses.

Fifty-one random, unrelated horses and three sire/dam/foal families were chosen for study in order to establish that a reasonable subset of the group of DNA markers found to date was likely to provide the desired p(exc) $\geq$ 0.90, and to assess the power of the DNA markers thereby allowing them to be prioritized for definitive allelic frequency measurements.

PCR generated single-stranded template DNA was prepared from the genomic DNA of each animal. This material was typed with respect to nucleotide variants using GBA. The genotype data obtained for each polymorphic site is summarized in Table 2. From this genotype data, allelic frequencies were determined and used to calculate the p(exc) of each site. The cumulative p(exc) is given for the group of 18 sites listed in Tables 1 and 2 is 0.955 for the group. In Tables 2-5, the genotype is indicated as either homozygote (i.e. PP or QQ) or the heterozygote (PQ). The numbers in parentheses denote the number of alleles of the genotype observed.

| TABLE 2 | | | | | | | | | |
|---|---|---|---|---|---|---|---|---|---|
| LOCUS | Genotype 1 PP (#) | Genotype 2 PQ (#) | Genotype 3 QQ (#) | p | q | p(exc) | p(non-exc) | cum p(non-exc) | cum p(exc) |
| 324-1 | CC (11) | CT (30) | TT (19) | 0.433 | 0.567 | 0.185 | 0.815 | 0.815 | 0.185 |
| 324-2 | CC (21) | CT (24) | TT ( 9) | 0.611 | 0.389 | 0.181 | 0.819 | 0.667 | 0.333 |
| 459-1 | AA ( 5) | AC (22) | CC (31) | 0.276 | 0.724 | 0.160 | 0.840 | 0.560 | 0.440 |
| 459-2 | CC (53) | CG ( 6) | GG ( 0) | 0.949 | 0.051 | 0.046 | 0.954 | 0.535 | 0.465 |
| 474-1 | AA (35) | AT (21) | TT ( 4) | 0.758 | 0.242 | 0.150 | 0.850 | 0.453 | 0.547 |
| 178-1 | AA (38) | AG (16) | GG ( 4) | 0.793 | 0.207 | 0.137 | 0.863 | 0.391 | 0.609 |
| 090-2 | AA (13) | AG (28) | GG (17) | 0.466 | 0.534 | 0.187 | 0.813 | 0.318 | 0.682 |
| 177-1 | AA ( 2) | AC (12) | CC (46) | 0.133 | 0.867 | 0.102 | 0.898 | 0.285 | 0.715 |
| 177-2 | CC (18) | CT (23) | TT (18) | 0.500 | 0.500 | 0.188 | 0.813 | 0.232 | 0.768 |
| 595-3 | AA (14) | AG (28) | GG (11) | 0.528 | 0.472 | 0.187 | 0.813 | 0.189 | 0.811 |
| 177-3 | AA (26) | AG (25) | GG ( 9) | 0.642 | 0.358 | 0.177 | 0.823 | 0.155 | 0.845 |
| 595-2 | GG (34) | GT (13) | TT ( 3) | 0.810 | 0.190 | 0.130 | 0.870 | 0.135 | 0.865 |
| 595-1 | AA (25) | AG (21) | GG ( 5) | 0.696 | 0.304 | 0.167 | 0.833 | 0.113 | 0.887 |
| 085-1 | CC (32) | CG (24) | GG ( 4) | 0.733 | 0.267 | 0.157 | 0.843 | 0.095 | 0.905 |
| 129-1 | AA ( 7) | AT (33) | TT (20) | 0.392 | 0.608 | 0.181 | 0.819 | 0.078 | 0.922 |
| 007-1 | AA (22) | CG (29) | GG ( 9) | 0.608 | 0.392 | 0.181 | 0.819 | 0.064 | 0.936 |
| 007-2 | AA ( 3) | AG (25) | GG (31) | 0.263 | 0.737 | 0.156 | 0.844 | 0.054 | 0.946 |
| 007-3 | AA (27) | AG (32) | GG ( 1) | 0.717 | 0.283 | 0.162 | 0.838 | 0.045 | 0.955 |

EP 0 726 905 B1

EXAMPLE 4

PARENTAGE TESTING

A family consisting of a sire, dam and offspring was typed with respect to the 18 variable sites discussed above with no exclusions found. This family had not been previously blood typed. Using the preliminary allelic frequency numbers given in Table 2, it is possible to construct a p(exc) table pertaining to this specific case (Table 3). In general, this Table is constructed assuming that the identity of the dam is not in question (although in practice, it is possible to exclude the mare if neither of her alleles is inherited by the foal). Table 3 shows the typing data for the foal and its dam with the sites tested listed in order of informativeness in this case. The overall cum p(exc) using 18 loci was 0.942.

TABLE 3

| LOCUS | FOAL | DAM | EXCLUDED SIRES | p(exc) | p(non-exc) | cum p(non-exc) | cum p(exc) |
|---|---|---|---|---|---|---|---|
| 459-1 | AC | **CC** | AA | 0.524 | 0.476 | 0.476 | 0.524 |
| 129-1 | AA | AT | TT | 0.370 | 0.630 | 0.300 | 0.700 |
| 324-1 | **CC** | CT | TT | 0.321 | 0.679 | 0.204 | 0.796 |
| 595-3 | GG | GG | AA | 0.279 | 0.721 | 0.147 | 0.853 |
| 090-2 | GG | AG | AA | 0.217 | 0.783 | 0.115 | 0.885 |
| 324-2 | CC | CT | TT | 0.151 | 0.849 | 0.098 | 0.902 |
| 595-1 | AA | AA | GG | 0.092 | 0.818 | 0.080 | 0.920 |
| 007-3 | AA | AA | GG | 00.80 | 0.920 | 0.073 | 0.927 |
| 085-1 | CC | CC | GG | 0.071 | 0.929 | 0.068 | 0.932 |
| 474-1 | AA | AA | TT | 0.059 | 0.941 | 0.064 | 0.936 |
| 178-1 | AA | AG | GG | 0.043 | 0.957 | 0.061 | 0.939 |
| 595-2 | GG | GG | TT | 0.036 | 0.964 | 0.059 | 0.941 |
| 177-1 | CC | CC | AA | 0.018 | 0.982 | 0.058 | 0.942 |
| 459-2 | CC | CC | GG | 0.003 | 0.997 | 0.058 | 0.942 |
| 007-1 | CG | CG | - | 0.000 | 1.000 | 0.058 | 0.942 |
| 007-2 | AG | AG | - | 0.000 | 1.000 | 0.058 | 0.942 |
| 177-2 | CT | CT | - | 0.000 | 1.000 | 0.058 | 0.942 |
| 177-3 | AG | AG | - | 0.000 | 1.000 | 0.058 | 0.942 |

**EXAMPLE 5**

IDENTITY TESTING

It is of interest to make use of the population analysis group to derive preliminary information concerning other aspects of the marker panel. For example, using the allelic frequency data, it is possible to calculate a probability of identity [p(ID)] value for the 18 sites which is equal to $4.79 \times 10^{-7}$ or approximately 1 in 2.1 million. Thus, one would predict that none of the horses examined in the population group would have the same genotype and computer analysis of the genotype database revealed this to be the case. As shown in Table 4, the p(ID) reaches very small numbers with analysis of comparatively few loci. Using the top seven sites, the probability of two random animals having different genotypes is already 99.9%.

TABLE 4

| LOCUS | GENOTYPE 1 | | GENOTYPE 2 | | GENOTYPE 3 | | p | q | p(ID) | cum p(ID) |
|---|---|---|---|---|---|---|---|---|---|---|
| | PP | (#) | PQ | (#) | QQ | (#) | | | | |
| 177.2 | CC | (18) | CT | (23) | TT | (18) | 0.500 | 0.500 | 0.375 | 0.375 |

TABLE 4   (continued)

| LOCUS | GENOTYPE 1 | | GENOTYPE 2 | | GENOTYPE 3 | | p | q | p(ID) | cum p(ID) |
|---|---|---|---|---|---|---|---|---|---|---|
| | PP | (#) | PQ | (#) | QQ | (#) | | | | |
| 595-3 | AA | (14) | AG | (28) | GG | (11) | 0.528 | 0.472 | 0.376 | 0.141 |
| 090-2 | AA | (13) | AG | (28) | GG | (17) | 0.466 | 0.534 | 0.376 | 0.053 |
| 324-1 | CC | (11) | CT | (30) | TT | (19) | 0.433 | 0.567 | 0.380 | 0.020 |
| 129-1 | AA | ( 7) | AT | (33) | TT | (20) | 0.392 | 0.608 | 0.388 | 0.008 |
| 007-1 | AA | (22) | CG | (29) | GG | (9) | 0.608 | 0.392 | 0.388 | 0.003 |
| 324-2 | CC | (21) | CT | (24) | TT | ( 9) | 0.611 | 0.389 | 0.388 | 0.001 |
| 177-3 | AA | (26) | AG | (25) | GG | (9) | 0.642 | 0.358 | 0.397 | $4.67 \times 10^{-4}$ |
| 595-1 | AA | (25) | AG | (21) | GG | (5) | 0.696 | 0.304 | 0.422 | $1.97 \times 10^{-4}$ |
| 007-3 | AA | (27) | AG | (32) | GG | (1) | 0.717 | 0.283 | 0.435 | $8.57 \times 10^{-4}$ |
| 459-1 | AA | (5) | AC | (22) | CC | (31) | 0.276 | 0.724 | 0.440 | $3.77 \times 10^{-5}$ |
| 085-1 | CC | (32) | CG | (24) | GG | (4) | 0.733 | 0.267 | 0.447 | $1.68 \times 10^{-5}$ |
| 007-2 | AA | (3) | AG | (25) | GG | (31) | 0.263 | 0.737 | 0.430 | $7.58 \times 10^{-6}$ |
| 474-1 | AA | (35) | AT | (21) | TT | (4) | 0.758 | 0.242 | 0.468 | $3.55 \times 10^{-6}$ |
| 178-1 | AA | (38) | AG | (16) | GG | (4) | 0.793 | 0.207 | 0.505 | $1,79 \times 10^{-6}$ |
| 595-2 | GG | (34) | GT | (13) | TT | (3) | 0.810 | 0.190 | 0.527 | $9.45 \times 10^{-7}$ |
| 177-1 | AA | (2) | AC | (12) | CC | (46) | 0.133 | 0.867 | 0.618 | $5,84 \times 10^{-7}$ |
| 459-2 | CC | (53) | CG | (6) | GG | (0) | 0.949 | 0.051 | 0.821 | $4.79 \times 10^{-7}$ |

<u>False Report Rate</u>

In the current study, two types of potential false reports can be encountered due to either (1) PCR failures or (2) incompatibility between the genotype obtained on opposite strands. Only data from those animals which had been successfully typed in both strands was included in the allelic frequency calculations. Sixty horses typed with respect to 18 sites amounts to 1,080 genotypings. 95% of all typing experiments were successful overall. No typing errors were due to traditional PCR failures. 3.8% false reports were encountered at the GBA step either because the PCR was unsuccessful at the single strand step or due to operator error. 1.1% of all typings produced incompatible data between the strands for unknown reasons.

In sum, the GBA (genetic bit analysis) method is thus a simple, convenient, and automatable method for interrogating SNPs. In this method, sequence-specific annealing to a solid phase-bound primer is used to select a unique polymorphic site in a nucleic acid sample, and interrogation of this site is via a highly accurate DNA polymerase reaction using a set of novel non-radioactive dideoxynucleotide analogs. One of the most attractive features of the GBA approach is that, because the actual allelic discrimination is carried out by the DNA polymerase, one set of reaction conditions can be used to interrogate many different polymorphic loci. This feature permits cost reductions in complex DNA tests by exploitation of parallel formats and provides for rapid development of new tests.

The intrinsic error rate of the GBA procedure in its present format is believed to be low; the signal-to-noise ratio in terms of correct vs. incorrect nucleotide incorporation for homozygotes appears to be approximately 20:1. GBA is thus sufficiently quantitative to allow the reliable detection of heterozygotes in genotyping studies. The presence in the DNA polymerase-mediated extension reaction of all four dideoxynucleoside triphosphates as the sole nucleotide substrates heightens the fidelity of genotype determinations by suppressing misincorporation. GBA can be used in any application where point mutation analyses are presently employed -- including genetic mapping and linkage studies, genetic diagnoses, and identity/paternity testing -- assuming that the surrounding DNA sequence is known.

### EXAMPLE 6

ANALYSIS OF A HUMAN SNP

Human single nucleotide polymorphisms may be used in the same manner as the above-described equine polymorphisms. Examples of suitable human polymorphisms are presented in Table 5.

EP 0 726 905 B1

| | | | TABLE 5 | | | | |
|---|---|---|---|---|---|---|---|
| | | | EXAMPLES OF HUMAN SINGLE NUCLEOTIDE POLYMORPHISMS | | | | |
| LOCUS | LOCATION | SEQ ID NO. | 5' PROXIMAL SEQUENCE | SNP ALLELE 1 | SNP ALLELE 2 | 3' DISTAL SEQUENCE | SEQ ID NO. |
| IGKC | 2p12 | 73 | AAAGCAGACTACGAGAAACACAAA | G | C | TCTACGCCTGCGAACTCACCCATC | 74 |
| | | 75 | GATGGGTGACTTCGCAGGCGTAGA | C | G | TTTGTGTTTCTCGTAGTCTCTTT | 76 |
| ILIB | 2q3-q21 | 77 | CTCCTGCAATTGACAGAGAGCTCC | C | T | GAGGCAGAGAACAGCACCCAAGCT | 78 |
| | | 79 | ACCTTGGGTGCTGTTCTCTGCCTC | G | A | GGAGCTCTCTGTCAATTGCAGGAG | 80 |
| LDLR | 19p13.3 | 81 | CTCCATCTCAAGCATCGATGTCAA | T | C | GGGGGCAACCGGAAGACCATCTTG | 82 |
| | | 83 | CAAGATGGTCTTCCGGTTGCCCCC | A | G | TTGACATCGATGCTTGAGATGGAG | 84 |
| MET-II | 7q31 | 85 | GTTTGGTCTAAGTTGCTGATTACC | A | G | GGATTTTTCTGACGATCTTTCAAC | 86 |
| | | 87 | GTTGAAAGATCGTCAGAAAAATCC | T | C | GGTAATCAGCAACTTAGACCAAAC | 88 |
| PROC | 2q13-q21 | 89 | GCTGACAGCGGCCCACTGCATGGA | T | C | GAGTCCAAGAAGCTCCTTGTCAGG | 90 |
| | | 91 | CCTGACAAGGAGCTTCTTGGACTC | A | G | TCCATGCAGTGGGCCGCTGTCAGC | 92 |

For the purpose of validating the strategy of converting human SNPs to a GBA test format, a phenotypically neutral SNP site was converted and tested by GBA. This site was selected from the Johns Hopkins University OMB database of human polymorphisms. The site is met-H on chromosome 7 at q31, mutation position 127, A to G (Horn, G.T. *et al.,* Clin. Chem. 36, 1614-1619, 1990). The following oligonucleotides were synthesized (p=phosphorothioate):

### PCR primer no. 1552 (SEQ ID NO:93)
5'-CpApTpCpCATGTAGGAGAGCCTTAGTC

### PCR primer no. 1553 (SEQ ID NO:94)
5'-CCATTTTTGTGTCTTCTAGTCTAAGG

### GBA primer no. 1554 (SEQ ID NO:95)
5'-TTGAAAGATCGTCAGAAAAATCC

Human DNA samples were randomly selected from the DNA archives of two families available from the Centre D'Etude du Polymorphisme Humaine (CEPH) family collection. A negative control, containing no DNA was also used. Sample DNAs were amplified by PCR using the above primers and the resulting product was analyzed by GBA for two potential bases at the polymorphic site, G and A. GBA results were obtained by an endpoint reading of absorbance at 450 nm in a microtiter plate reader. The data is presented in Table 6.

Samples 1, 2, 4, 6 and 8 were homozygous for A, samples 7 and 9 were homozygous for G and samples 3 and 5 were GA heterozygotes. These DNAs have not been tested for this biallelism by any other method to date.

TABLE 6

| Sample No. | CEPH DNA No. | Adsorption at A$_{450}$ | | Genotype |
|------------|--------------|----------|----------|----------|
|            |              | Base G | Base A |          |
| 1 | 1333-10 | .100 | .556 | AA |
| 2 | 1333-02 | .084 | .782 | AA |
| 3 | 1333-04 | .372 | .369 | GA |
| 4 | 1333-05 | .081 | .905 | AA |
| 5 | 1333-07 | .321 | .346 | GA |
| 6 | 1333-08 | .084 | .803 | AA |
| 7 | 1340-09 | .675 | .092 | GG |
| 8 | 1340-10 | .084 | .756 | AA |
| 9 | 1340-12 | .537 | .096 | GG |
| No DNA | N/A | .076 | .097 | N/A |

### False Report Rate

In the current study, two types of potential false reports can be encountered due to either (1) PCR failures or (2) incompatibility between the genotype obtained on opposite strands. Only data from those animals which had been successfully typed in both strands was included in the allelic frequency calculations. Sixty horses typed with respect to 18 sites amounts to 1,080 genotypings. 95% of all typing experiments were successful overall. No typing errors were due to traditional PCR failures. 3.8% false reports were encountered at the GBA step either because the PCR was unsuccessful at the single strand step or due to operator error. 1.1% of all typings produced incompatible data between the strands for unknown reasons.

In sum, the GBA (genetic bit analysis) method is a simple, convenient, and automatable method for interrogating SNPs. In this method, sequence-specific annealing to a solid phase-bound primer is used to select a unique polymorphic site in a nucleic acid sample, and interrogation of this site is via a highly accurate DNA polymerase reaction using a set of novel non-radioactive dideoxynucleotide analogs. One of the most attractive features of the GBA approach is that, because the actual allelic discrimination is carried out by the DNA polymerase, one set of reaction conditions can be used to interrogate many different polymorphic loci. This feature permits cost reductions in complex DNA tests by exploitation of parallel formats and provides for rapid development of new tests.

The intrinsic error rate of the GBA procedure in its present format is believed to be low; the signal-to-noise ratio in terms of correct vs. incorrect nucleotide incorporation. for homozygotes appears to be approximately 20:1. GBA is thus sufficiency quantitative to allow the reliable detection of heterozygotes in genotyping studies. The presence in the DNA polymerase-mediated extension reaction of all four dideoxynucleoside triphosphates as the sole nucleotide substrates heightens the fidelity of genotype determinations by suppressing misincorporation. GBA can be used in any application where point mutation analyses are presently employed -- including genetic mapping and linkage studies, genetic diagnoses, and identity/paternity testing -- assuming that the local surrounding DNA sequence is known.

**Claims**

1. A method for genetic analysis of a set of individuals of the same species comprising:

providing a polymorphic array comprising a set of single nucleotide polymorphisms (SNPs); and determining the presence or absence of the polymorphisms in the set of SNPs in each of the set of individuals; and

determining whether the presence or absence of a particular allele of a polymorphism in the set of SNPs is associated with a particular trait.

2. A method according to claim 1, wherein the SNP does not cause the trait.

3. A method according to claim 1 further comprising:

analysing the frequency of segregation between the SNPs in the set, thereby establishing a genetic map in which the SNPs act as markers.

4. A method of determining the probability that a nucleic acid sample is derived from a particular individual comprising:

   providing a polymorphic array comprising a set of single nucleotide polymorphisms (SNPs) from said individual and a corresponding polymorphic array from said sample;
   determining the presence or absence of multiple SNP markers in the two arrays and comparing the results for each SNP marker;
   determining therefrom a probability of identity or non-identity from each comparison; and
   determining therefrom a cumulative probability of identity or non-identity by multiplying the probabilities provided by each comparison.

5. A method according to claim 4, wherein the nucleic acid sample is unknown and the particular individual is known.

6. A method according to claim 4, wherein the polymorphic array comprises a reference set of genetic markers comprising three or more SNPs.

7. A method according to claim 4, wherein comparing the results for each SNP marker includes determining the allelic frequency of the SNPs.

8. A method according to claim 4, wherein the cumulative probability of identity or non-identity is greater than about 0.95.

9. A method according to claim 4, wherein the nucleic acid molecules are DNA.

10. A method according to claim 4, wherein the nucleic acid molecules are RNA.

11. A method of determining the likelihood that an individual is or is not the progeny of a putative ancestor or ancestors comprising:

   providing a polymorphic array comprising a set of single nucleotide polymorphisms (SNPs) from said individual and a corresponding polymorphic array from said putative ancestor or ancestors;
   determining the presence or absence of multiple SNP markers in the individual's array and the putative ancestor's or ancestors' and comparing the results for each SNP marker; and
   determining therefrom the likelihood that the individual is or is not the progeny of the putative ancestor or ancestors.

12. A method according to claim 11, wherein the putative ancestor is a putative parent, or the putative ancestors are putative parents.

13. A method according to claim 11, wherein the method is used to exclude paternity of a putative male parent for the individual by calculation of a probability of paternity exclusion from each comparison, and determining therefrom a cumulative probability of paternity exclusion by multiplying said probabilities of paternity exclusion.

14. A method according to claim 11, wherein the method is used to assess the probability that the individual is the progeny of a selected putative female parent.

15. A method according to claim 11, wherein the cumulative probability of identity or non-identity is 0.95 or greater.

16. A method according to claim 12 or 13, wherein the cumulative probability of exclusion is 0.95 or greater.

17. A method according to claim 16, wherein the probability of exclusion is greater than 0.99.

18. A method according to claim 11, wherein determining the likelihood that the individual is or is not the progeny of the putative ancestor or ancestors is achieved by identifying matches obtained by comparing the results for each SNP marker and detecting allelic frequencies of the SNPs in the polymorphic array or arrays.

19. A method according to any one of the preceding claims, wherein the SNP markers are at multiple unlinked loci.

20. A method according to any one of the preceding claims, wherein the SNPs are diallelic or trialletic.

21. A method according to any one of the preceding claims which is carried out for the purpose of genotyping a plant or animal.

22. A method according to claim 21, wherein the animal is a murine, human, ovine, equine, bovine, porcine, canine or feline animal.

23. A method according to claim 1, wherein the trait is a predisposition to a genetic disease.

24. A method according to any one of the preceding claims, wherein the presence or absence of the SNPs is determined by Genetic Bit Analysis (GBA).

25. A method according to any one of the preceding claims, wherein the polymorphic array comprises 3 or more SNPs.

26. A method of generating a genetic map of an individual, comprising:

(a) providing a polymorphic array comprising three or more single nucleotide polymorphisms (SNPs);
(b) identifying the SNP variants present in an ancestor of the individual by determining the base identity at each SNP site of the ancestor of the individual and identifying the SNP variants present in the individual by determining the base identity at each SNP site of the individual;
(c) determining the number of matches between the individual and the ancestor; and
(d) calculating the extent of genetic linkage between each allele from the number of matches of step (c) and the probability that any pair of alleles found in the individual were inherited from the same ancestor based on the allelic frequencies of the SNP variants of the polymorphic array, thereby generating the genetic map of the individual.

27. A method according to claim 26, wherein the SNP sites used to construct the genetic map are randomly distributed throughout the genome of the species.

28. A method according to claim 26, wherein the ancestor is selected from the group consisting of parent and grandparent.

29. A method according to claim 26, wherein the SNP variants do not cause a genetic trait.

30. A method according to claim 1, wherein the trait of interest is a predisposition to a genetic disease.

31. A method according to claim 1, wherein the trait of interest is a genetic disease.

32. A method according to claim 1, wherein the individual is selected from an animal and a plant.

33. A method according to claim 32, wherein the individual is a mammal.

34. A method according to claim 33, wherein the mammal is selected from humans, non-human primates, dogs, cats, cattle, sheep, horses, mouse, rat and rabbit.

35. A method according to claim 34, wherein the mammal is a human.

36. A method according to claim 34, wherein the mammal is a horse.

37. A method according to claim 1, wherein each SNP has an allelic frequency of at least 0.20.

38. A method according to claim 1, further comprising calculating a LOD score and establishing a genetic linkage of SNP variants and the trait of interest.

39. A method according to claim 1, wherein the polymorphic array comprises three or more SNPs.

40. A method according to claim 1, wherein none of the SNP variants in the polymorphic array cause the trait.

**EP 0 726 905 B1**

**Patentansprüche**

1. Verfahren zur genetischen Analyse eines Satzes von Individuen derselben Spezies, aufweisend:

   Bereitstellen einer polymorphen Gruppe, die einen Satz von Einzelnucleotidpolymorphismen (SNPs) aufweist; und

   Feststellen des Vorliegens oder Fehlens der Polymorphismen in dem Satz von SNPs bei jedem des Satzes von Individuen; und

   Feststellen, ob des Vorliegen oder Fehlen eines bestimmten Allels eines Polymorphismus in dem Satz von SNPs mit einer bestimmten Eigenschaft verbunden ist.

2. Verfahren nach Anspruch 1, bei dem der SNP nicht die Eigenschaft bewirkt.

3. Verfahren nach Anspruch 1, außerdem aufweisend:

   Analysieren der Weitergabehäufigkeit zwischen den SNPs in dem Satz unter Erstellen einer Genkarte, in der die SNPs als Marker wirken.

4. Verfahren zur Bestimmung der Wahrscheinlichkeit, dass eine Nucleinsäure-Probe von einem bestimmten Individuum stammt, aufweisend:

   Bereitstellen einer polymorphen Gruppe, die einen Satz von Einzelnucleotidpolymorphismen (SNPs) von dem Individuum und eine entsprechende polymorphe Gruppe von der Probe aufweist;

   Feststellen des Vorliegens oder Fehlens mehrerer SNP-Marker in den zwei Gruppen und Vergleichen der Ergebnisse für jeden SNP-Marker;

   davon ausgehend Bestimmen einer Wahrscheinlichkeit der Identität oder Nicht-Identität aus jedem Vergleich; und

   davon ausgehend Bestimmen einer kumulativen Wahrscheinlichkeit der Identität oder Nicht-Identität durch Multiplizieren der von jedem Vergleich gelieferten Wahrscheinlichkeiten.

5. Verfahren nach Anspruch 4, bei dem die Nucleinsäure-Probe unbekannt ist und das bestimmte Individuum bekannt ist.

6. Verfahren nach Anspruch 4, bei dem die polymorphe Gruppe einen drei oder mehr SNPs aufweisenden Referenzsatz von Genmarkern aufweist.

7. Verfahren nach Anspruch 4, bei dem das Vergleichen der Ergebnisse für jeden SNP-Marker das Bestimmen der Allelhäufigkeit der SNPs beinhaltet.

8. Verfahren nach Anspruch 4, bei dem die kumulative Wahrscheinlichkeit der Identität oder Nicht-Identität größer als etwa 0,95 ist.

9. Verfahren nach Anspruch 4, bei dem die Nucleinsäure-Moleküle DNA sind.

10. Verfahren nach Anspruch 4, bei dem die Nucleinsäure-Moleküle RNA sind.

11. Verfahren zur Bestimmung der Wahrscheinlichkeit, dass ein Individuum der Nachkomme eines mutmaßlichen Vorfahren oder von mutmaßlichen Vorfahren ist oder nicht ist, aufweisend:

   Bereitstellen einer polymorphen Gruppe, die einen Satz von Einzelnucleotidpolymorphismen (SNPs) von dem Individuum und eine entsprechende polymorphe Gruppe von dem mutmaßlichen Vorfahren oder den mutmaßlichen Vorfahren aufweist;

Feststellen des Vorliegens oder Fehlens mehrerer SNP-Marker in den Gruppen des Individuums und des mutmaßlichen Vorfahren oder der mutmaßlichen Vorfahren und Vergleichen der Ergebnisse für jeden SNP-Marker; und

davon ausgehend Bestimmen der Wahrscheinlichkeit, dass das Individuum der Nachkomme des mutmaßlichen Vorfahren oder der mutmaßlichen Vorfahren ist oder nicht ist.

12. Verfahren nach Anspruch 11, bei dem der mutmaßliche Vorfahr ein mutmaßlicher Elternteil ist oder die mutmaßlichen Vorfahren mutmaßliche-Eltern sind.

13. Verfahren nach Anspruch 11, bei dem das Verfahren zum Ausschließen der Vaterschaft eines mutmaßlichen männlichen Elternteils für das Individuum durch Berechnung einer Wahrscheinlichkeit des Vaterschaftsausschlusses aus jedem Vergleich und durch daraus Bestimmen einer kumulativen Wahrscheinlichkeit des Vaterschaftsausschlusses durch Multiplizieren der Wahrscheinlichkeiten des Vaterschaftsauschlusses verwendet wird.

14. Verfahren nach Anspruch 11, bei dem das Verfahren zur Beurteilung der Wahrscheinlichkeit, dass das Individuum der Nachkomme eines ausgewählten mutmaßlichen weiblichen Elternteils ist, verwendet wird.

15. Verfahren nach Anspruch 11, bei dem die kumulative Wahrscheinlichkeit der Identität oder Nicht-Identität 0,95 oder größer ist.

16. Verfahren nach Anspruch 12 oder 13; bei dem die kumulative Wahrscheinlichkeit des Ausschlusses 0,95 oder größer ist.

17. Verfahren nach Anspruch 16, bei dem die Wahrscheinlichkeit des Ausschlusses größer als 0,99 ist.

18. Verfahren nach Anspruch 11, bei dem die Bestimmung der Wahrscheinlichkeit, dass das Individuum der Nachkomme des mutmaßlichen Vorfahren oder der mutmaßlichen Vorfahren ist oder nicht ist, erreicht wird durch Identifizieren von Gegenstücken, die erhalten wurden durch Vergleichen der Ergebnisse für jeden SNP-Marker und Bestimmen von Allelhäufigkeiten der SNPs in der polymorphen Gruppe oder den polymorphen Gruppen.

19. Verfahren nach einem der vorangehenden Ansprüche, bei dem die SNP-Marker an mehreren unverbundenen Orten sind.

20. Verfahren nach einem der vorangehenden Ansprüche, bei dem die SNPs dialtelisch oder triallelisch sind.

21. Verfahren nach einem der vorangehenden Ansprüche, das zum Zweck der Genotypisierung einer Pflanze oder eines Tieres durchgeführt wird.

22. Verfahren nach Anspruch 21, bei dem das Tier eine Maus, ein Mensch, ein Schaf, ein Pferd, ein Rind, ein Schwein, ein Hund oder eine Katze ist.

23. Verfahren nach Anspruch 1, bei dem die Eigenschaft eine Anfälligkeit für eine genetische Krankheit ist.

24. Verfahren nach einem der vorangehenden Ansprüche, bei dem das Vorliegen oder Fehlen der SNPs durch genetische Bitanalyse (GBA) festgestellt wird.

25. Verfahren nach einem der vorangehenden Ansprüche, bei dem die polymorphe Gruppe drei oder mehr SNPs aufweist.

26. Verfahren zur Erzeugung einer Genkarte eines Individuums, aufweisend:

(a) Bereitstellen einer polymorphen Gruppe, die drei oder mehr Einzelnucleotidpolymorphismen (SNPs) aufweist;

(b) Identifizieren der bei einem Vorfahren des Individuums vorliegenden SNP-Varianten durch Bestimmen der Basenidentität an jeder SNP-Stelle des Vorfahren des Individuums und Identifizieren der bei dem Individuum vorliegenden SNP-Varianten durch Bestimmen der Basenidentität an jeder SNP-Stelle des Individuums;

(c) Bestimmen der Anzahl von Gegenstücken zwischen dem Individuum und dem Vorfahren; und

(d) Berechnen des Ausmaßes der genetischen Verknüpfung zwischen jedem Allel aus der Anzahl von Gegenstücken aus Schritt (c) und der Wahrscheinlichkeit, dass irgendein bei dem Individuum gefundenes Paar von Allelen von demselben Vorfahren geerbt wurde, auf der Basis der Allelhäufigkeiten der SNP-Varianten der polymorphen Gruppe, wodurch die Genkarte des Individuums erstellt wird.

27. Verfahren nach Anspruch 26, bei dem die zum Aufbau der Genkarte verwendeten SNP-Stellen willkürlich überall in dem Genom der Spezies verteilt sind.

28. Verfahren nach Anspruch 26, bei dem der Vorfahr ausgewählt wird aus der Gruppe, die aus Eltern und Großeltern besteht.

29. Verfahren nach Anspruch 26, bei dem die SNP-Varianten keine genetische Eigenschaft bewirken.

30. Verfahren nach Anspruch 1, bei dem die Eigenschaft von Interesse eine Anfälligkeit für eine genetische Krankheit ist.

31. Verfahren nach Anspruch 1, bei dem die Eigenschaft von Interesse eine genetische Krankheit ist.

32. Verfahren nach Anspruch 1, bei dem das Individuum aus einem Tier und einer Pflanze ausgewählt wird.

33. Verfahren nach Anspruch 32, bei dem das Individuum ein Säugetier ist.

34. Verfahren nach Anspruch 33, bei dem das Säugetier ausgewählt wird aus Menschen, nicht-menschlichen Primaten, Hunden, Katzen, Rindern, Schafen, Pferden, Mäusen, Ratten und Kaninchen.

35. Verfahren nach Anspruch 34, bei dem das Säugetier ein Mensch ist.

36. Verfahren nach Anspruch 34, bei das Säugetier ein Pferd ist.

37. Verfahren nach Anspruch 1, bei dem jeder SNP eine Allelhäufigkeit von mindestens 0,20 hat.

38. Verfahren nach Anspruch 1, außerdem aufweisend die Berechnung eines LOD-Werts und das Feststellen einer genetischen Verknüpfung von SNP-Varianten und der Eigenschaft von Interesse.

39. Verfahren nach Anspruch 1, bei dem die polymorphe Gruppe drei oder mehr SNPs aufweist.

40. Verfahren nach Anspruch 1, bei dem keine der SNP-Varianten in der polymorphen Gruppe die Eigenschaft bewirkt.

**Revendications**

1. Procédé d'analyse génétique d'un ensemble d'individus de la même espèce dans lequel:

   on fournit une série de polymorphismes comprenant un jeu de polymorphismes d'un seul nucléotide (SNP); et
   on détermine la présence ou l'absence des polymorphismes dans le jeu de SNP dans chacun des ensembles d'individus; et
   on détermine si la présence ou l'absence d'un allèle particulier d'un polymorphisme dans le jeu de SNP est associée à un caractère particulier.

2. Procédé selon la revendication 1, dans lequel le SNP ne donne pas le caractère.

3. Procédé selon la revendication 1, dans lequel, en outre :

   on analyse la fréquence de ségrégation entre les SNP dans le jeu, en établissant ainsi une carte génétique dans laquelle les SNP jouent le rôle de marqueurs.

**4.** Procédé de détermination de la probabilité selon laquelle un échantillon d'acides nucléiques provient d'un individu particulier, dans lequel:

on fournit une série de polymorphismes comprenant un jeu de polymorphismes d'un seul nucléotide (SNP) dudit individu et une série de polymorphismes correspondante dudit échantillon;
on détermine la présence ou l'absence de SNP marqueurs multiples dans les deux séries et on compare les résultats pour chaque SNP marqueur;
on détermine une probabilité d'identité ou de non identité à partir de chaque comparaison; et
on détermine une probabilité cumulée d'identité ou de non identité en multipliant les probabilités fournies par chaque comparaison.

**5.** Procédé selon la revendication 4, dans lequel l'échantillon d'acides nucléiques est inconnu et l'individu particulier est connu.

**6.** Procédé selon la revendication 4, dans lequel la série de polymorphismes comprend un jeu de référence de marqueurs génétiques comprenant au moins 3 SNP.

**7.** Procédé selon la revendication 4, dans lequel la comparaison des résultats pour chaque SNP marqueur comprend la détermination de la fréquence allélique des SNP.

**8.** Procédé selon la revendication 4, dans lequel la probabilité cumulée d'identité ou de non identité est supérieure à environ 0,95.

**9.** Procédé selon la revendication 4, dans lequel les molécules d'acides nucléiques sont des ADN.

**10.** Procédé selon la revendication 4, dans lequel les molécules d'acides nucléiques sont des ARN.

**11.** Procédé de détermination de la probabilité selon laquelle un individu est ou n'est pas le descendant d'un ancêtre putatifs ou d'ancêtres putatifs, dans lequel:

on fournit une série de polymorphismes comprenant un jeu de polymorphismes d'un seul nucléotide (SNP) dudit individu et une série de polymorphismes correspondante dudit ou desdits ancêtres putatifs;
on détermine la présence ou l'absence de marqueurs SNP multiples dans la série de l'individu et dans celles du ou des ancêtres putatifs et on compare les résultats pour chaque marqueur SNP;
on en déduit la probabilité avec laquelle l'individu est ou n'est pas le descendant du ou des ancêtres putatifs.

**12.** Procédé selon la revendication 11, dans lequel l'ancêtre putatif est un parent putatif, ou les ancêtres putatifs sont les parents putatifs.

**13.** Procédé selon la revendication 11, dans lequel on utilise le procédé pour exclure la paternité d'un parent mâle putatif pour l'individu en calculant une probabilité d'exclusion de paternité à partir de chaque comparaison, et en déterminant à partir de là une probabilité cumulée d'exclusion de paternité par multiplication desdites probabilités d'exclusion de paternité.

**14.** Procédé selon la revendication 11, dans lequel on utilise le procédé pour évaluer la probabilité selon laquelle l'individu est le descendant d'un parent femelle putatif sélectionné.

**15.** Procédé selon la revendication 11, dans lequel la probabilité cumulée d'identité ou de non identité est d'au moins 0,95.

**16.** Procédé selon la revendication 12 ou 13, dans lequel la probabilité cumulée d'exclusion est d'au moins 0,95.

**17.** Procédé selon la revendication 16, dans lequel la probabilité d'exclusion est supérieure à 0,99.

**18.** Procédé selon la revendication 11, dans lequel on détermine la probabilité selon laquelle l'individu est ou non le descendant de l'ancêtre putatif ou des ancêtres putatifs en identifiant les appariements obtenus par comparaison des résultats pour chaque marqueur SNP et en détectant les fréquences alléliques des SNP dans la série ou les séries de polymorphismes.

**19.** Procédé selon l'une quelconque des revendications précédentes, dans lequel les marqueurs SNP sont à des locus multiples non liés.

**20.** Procédé selon l'une quelconque des revendications précédentes, dans lequel les SNP sont dialléliques ou trialléliques.

**21.** Procédé selon l'une quelconque des revendications précédentes, qui est effectué à des fins de génotypage d'une plante ou d'un animal.

**22.** Procédé selon la revendication 21, dans lequel l'animal est un animal murin, humain, ovin, équin, bovin, porcin, canin ou félin.

**23.** Procédé selon la revendication 1, dans lequel le caractère est une prédisposition à une maladie génétique.

**24.** Procédé selon l'une quelconque des revendications précédentes, dans lequel la présence ou l'absence des SNP est déterminée par analyse génétique binaire (GBA = *Genetic Bit Analysis*).

**25.** Procédé selon l'une quelconque des revendications précédentes, dans laquelle la série de polymorphismes comprend au moins 3 SNP.

**26.** Procédé de production d'une carte génétique d'un individu, dans lequel

(a) on fournit une série de polymorphismes comprenant au moins 3 polymorphismes d'un seul nucléotide (SNP);
(b) on identifie les variantes de SNP présentes chez un ancêtre de l'individu en déterminant l'identité de bases à chaque site de SNP de l'ancêtre de l'individu et on identifie les variantes de SNP présentes chez l'individu en déterminant l'identité de bases à chaque site de SNP de l'individu;
(c) on détermine le nombre d'appariements entre l'individu et l'ancêtre;
(d) on calcule l'étendue de liaison génétique entre chaque allèle à partir du nombre d'appariements de l'étape (c) et la probabilité selon laquelle les paires d'allèles trouvées chez l'individu ont été héritées du même ancêtre à partir des fréquences alléliques des variantes de SNP de la série de polymorphismes, grâce à quoi on produit la carte génétique de l'individu.

**27.** Procédé selon la revendication 26, dans lequel les sites de SNP utilisés pour la construction de la carte génétique sont répartis au hasard dans tout le génome de l'espèce.

**28.** Procédé selon la revendication 26, dans lequel l'ancêtre est choisi dans le groupe constitué par les parents et les grands-parents.

**29.** Procédé selon la revendication 26, dans lequel les variantes de SNP ne donnent pas un caractère génétique.

**30.** Procédé selon la revendication 1, dans lequel le caractère d'intérêt est une prédisposition à une maladie génétique.

**31.** Procédé selon la revendication 1, dans lequel le caractère d'intérêt est une maladie génétique.

**32.** Procédé selon la revendication 1, dans lequel l'individu est choisi parmi un animal et une plante.

**33.** Procédé selon la revendication 32, dans lequel l'individu est un mammifère.

**34.** Procédé selon la revendication 33, dans lequel le mammifère est choisi parmi l'homme, les primates non humains, le chien, le chat, le bétail, le mouton, le cheval, la souris, le rat et le lapin.

**35.** Procédé selon la revendication 34, dans lequel le mammifère est l'homme.

**36.** Procédé selon la revendication 34, dans lequel le mammifère est le cheval.

**37.** Procédé selon la revendication 1, dans lequel chaque SNP a une fréquence allélique d'au moins 0,20.

**38.** Procédé selon la revendication 1, comprenant en outre le calcul d'un score LOD et l'établissement d'une liaison génétique de variantes de SNP et du caractère d'intérêt.

**39.** Procédé selon la revendication 1, dans lequel la série de polymorphismes comprend au moins 3 SNP.

**40.** Procédé selon la revendication 1, dans lequel aucune des variantes de SNP dans la série de polymorphismes ne donne le caractère.

FIG. 1

FIG. 2A

#TB855

FIG. 2C

#TB853

FIG. 3

FIG. 4

FIG. 5

EP 0 726 905 B1

| | | TABLE 1 | | | |
|---|---|---|---|---|---|
| | | **POLYMORPHIC LOCI** | | | |
| CLONE NO. | SEQ ID NO. | 5' PROXIMAL SEQUENCE | SNP ALLELE 1  2 | 3' DISTAL SEQUENCE | SEQ ID NO. |
| 177-2 | 1 | GCAGCTCTAAGTGCTGTGGG | C   T | TGCAGAAATTCTAAGGTGTT | 2 |
| | 3 | AACACCTTAGAATTTCTGCA | G   A | CCCACAGCACTTAGAGCTGC | 4 |

5' → 3'
3' → 5'

ALLELE 1

5' GCAGCTCTAAGTGCTGTGGG   C TGCAGAAATTCTAAGGTGTT 3'
3' CGTCGAGATTCACGACACCC   G ACGTCTTTAAGATTCCACAA 5'

ALLELE 2

5' GCAGCTCTAAGTGCTGTGGG   T TGCAGAAATTCTAAGGTGTT 3'
3' CGTCGAGATTCACGACACCC   A ACGTCTTTAAGATTCCACAA 5'

FIG.6

FIG.7

FIG.8A

FIG.8B

SEQUENCE LISTING

(1) GENERAL INFORMATION:

(i) APPLICANT:

(A) NAME: ORCHID BIOSCIENCES, INC.

(i) TITLE OF INVENTION: SINGLE NUCLEOTIDE POLYMORPHISMS AND THEIR USE IN GENETIC ANALYSIS

(iii) NUMBER OF SEQUENCES: 95

(iv) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: Patentln Release #1.0, Version #1.25

(v) CURRENT APPLICATION DATA:
     APPLICATION NUMBER: EP 95900520.8

(2) INFORMATION FOR SEQ ID NO:1:

(i) SEQUENCE CHARACTERISTICS:

(A) LENGTH: 20 base pairs.
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus-caballus

(vii) IMMEDIATE SOURCE:

    (B) CLONE: 177-2

(xi) SEQUENCE DESCRIPTION: SEQ ID NO:1:

        GCAGCTCTAA GTGCTGTGGG                    20

(2) INFORMATION FOR SEQ ID NO:2:

    (i) SEQUENCE CHARACTERISTICS:

        (A) LENGTH: 20 base pairs
        (B) TYPE: nucleic add
        (C) STRANDEDNESS: single
        (D) TOPOLOGY: linear

    (ii) MOLECULE TYPE: DNA (genomic)

    (iii) HYPOTHETICAL NO

    (iv) ANTI-SENSE: NO

    (vi) ORIGINAL SOURCE:

        (A) ORGANISM: Equus caballus

    (vii) IMMEDIATE SOURCE:

        (B) CLONE: 177-2

    (xi) SEQUENCE DESCRIPTION: SEO ID NO:2:

        TGCAGAAATT CTAAGGTGTT                    20

(2) INFORMATION FOR SEQ ID NO:3:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

(B) CLONE: 177-2

(xi) SEQUENCE DESCRIPTION:SEQ ID NO:3:

AACACCTTAG AATTTCTGCA                                             20

(2) INFORMATION FOR SEQ ID NO:4:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 177-2

(xi) SEQUENCE DESCRIPTION: SEQ ID NO:4:

CCCACAGCAC TTAGAGCTGC                                             20

(2) INFORMATION FOR SEQ ID NO:5:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-3

(xi) SEQUENCE DESCRIPTION: SEQ ID NO:5:

**AGCTCTGGGA TGATCCACTA** 20

(2) INFORMATION FOR SEQ ID NO:6:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-3

(xi) SEQUENCE DESCRIPTION: SEQ ID NO:6:

**TGAGGGAAAA ATGATGATGC** 20

(2) INFORMATION FOR SEQ ID NO:7:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-3

(xi) SEQUENCE DESCRIPTION: SEQ ID NO:7:

**GCATCATCAT TTTTCCCTCA**            **20**

(2) INFORMATION FOR SEQ ID NO:8:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 595-3

    (xi) SEQUENCE DESCRIPTION: SEQ ID NO:8:

**TAGTGGATCA TCCCAGAGCT**            **20**

(2) INFORMATION FOR SEQ ID NO:9:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 090-2

    (xi) SEQUENCE DESCRIPTION: SEQ ID NO:9:

AAAACTAATT TGATGGCCAT 20

(2) INFORMATION FOR SEQ ID NO:10:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 090-2

(xi) SEQUENCE DESCRIPTION: SEQ ID NO:10:

AAAGTCAGAA CAATGATTGC 20

(2) INFORMATION FOR SEQ ID NO:11:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 090-2

(xi) SEQUENCE DESCRIPTION: SEQ ID NO:11:

GCAATCATTG TTCTGACTTT                20

(2) INFORMATION FOR SEQ ID NO:12:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 090-2

    (xi) SEQUENCE DESCRIPTION: SEQ ID NO:12:

ATGGCCATCA AATTAGTTTT                20

(2) INFORMATION FOR SEQ ID NO:13:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 324-1

    (xi) SEQUENCE DESCRIPTION: SEQ ID NO:13

CACAAGGCCC AAGAACAGGA                20

(2) INFORMATION FOR SEQ ID NO:14:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 324-1

(xi) SEQUENCE DESCRIPTION: SEQ ID NO:14:

TGAGTTCAGC GAGTGTCAGA                                    20

(2) INFORMATION FOR SEQ ID NO:15:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 324-1

(xi) SEQUENCE DESCRIPTION: SEQ ID NO,15:

TCTGACACTC GCTGAACTCA                                    20

(2) INFORMATION FOR SEQ ID NO: 16:

EP 0 726 905 B1

(i) SEQUENCE CHARACTERISTICS:

(A) LENGTH: 20 base pairs
(B) TYPE: nucleic add
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 324-1

(xi) SEQUENCE DESCRIPTION: SEQ ID NO:16:

TCCTGTTCTT GGGCCTTGTG                                                    20

(2) INFORMATION FOR SEQ ID NO:17:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 129-1

(xi) SEQUENCE DESCRIPTION: SEQ ID NO:17:

TGGGAAAGAC CACATTATTT                                                    20

(2) INFORMATION FOR SEQ ID NO:18:

(i) SEQUENCE CHARACTERISTICS:

36

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 129-1

(xi) SEQUENCE DESCRIPTION: SEQ ID NO:18:

        **GTTCCCTTTT GTTTCAGACC**         **20**

(2) INFORMATION FOR SEQ ID NO:19:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 129-1

    (xi) SEQUENCE DESCRIPTION: SEO ID NO-1 9:

        **GGTCTGAAAC AAAAGGGAAC**         **20**

(2) INFORMATION FOR SEO ID NO:20:

    (i) SEQUENCE CHARACTERISTICS:

        (A) LENGTH: 20 base pairs
        (B) TYPE: nucleic acid

(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 129-1

(xi) SEQUENCE DESCRIPTION: SEQ ID NO:20:

AAATAATGTG GTCTTTCCCA                                20

(2) INFORMATION FOR SEQ ID NO:21:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 007-1

(xi) SEQUENCE DESCRIPTION: SEQ ID NO:21:

CATGAGTAAG AAGCATCCGG                                20

(2) INFORMATION FOR SEQ ID NO:22:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 007-1

(xi) SEQUENCE DESCRIPTION: SEQ ID NO:22:

          CCATGGAGTC ATAGATAAGT               20

(2) INFORMATION FOR SEQ ID NO:23:

(i) SEQUENCE CHARACTERISTICS:

    (A) LENGTH: 20 base pairs
    (B) TYPE: nucleic add
    (C) STRANDEDNESS: single
    (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 007-1

(xi) SEQUENCE DESCRIPTION: SEQ ID NO:23:

          ACTTATCTAT GACTCCATGG               20

(2) INFORMATION FOR SEQ ID NO:24:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 007-1

(xi) SEQUENCE DESCRIPTION: SEQ ID NO:24:

        CCGGATGCTT CTTACTCATG                 20

(2) INFORMATION FOR SEQ ID NO:25:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 324-2

    (xi) SEQUENCE DESCRIPTION: SEQ ID NO:25:

        CCCAAGAACA GGATTGAGTT                 20

(2) INFORMATION FOR SEQ ID NO:26:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 324-2

(xi) SEQUENCE DESCRIPTION: SEQ ID NO-26:

        AGCGAGTGTC AGAGTTGTGT          20

(2) INFORMATION FOR SEQ ID NO:27:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 324-2

    (xi) SEQUENCE DESCRIPTION: SEQ ID NO:27:

        ACACAACTCT GACACTCGCT          20

(2) INFORMATION FOR SEQ ID NO:28:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

(vi) ORIGINAL SOURCE:

    (A) ORGANISM: Equus caballus

(vii) IMMEDIATE SOURCE:

    (B) CLONE: 324-2

(xi) SEQUENCE DESCRIPTION: SEQ ID NO:28:

          AACTCAATCC TGTTCTTGGG                 20

(2) INFORMATION FOR SEQ ID NO:29:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus cabailus

    (vii) IMMEDIATE SOURCE:

        (B) CLONE: 177.3

    (xi) SEQUENCE DESCRIPTION: SEQ ID NO:29:

          AGCAAGAAA TGGGGGGCCTT                20

(2) INFORMATION FOR SEQ ID NO:30:

    (i) SEQUENCE CHARACTERISTICS:

        (A) LENGTH: 20 base pairs
        (B) TYPE: nucleic add
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

(A) ORGANISM: Equus caballus

(vii) IMMEDIATE SOURCE:

(B) CLONE: 177-3

(xi) SEQUENCE DESCRIPTION: SEQ ID NO:30:


GTCCTACAAT TGCCAGGAAG                    20


(2) INFORMATION FOR SEQ ID NO:

(i) SEQUENCE CHARACTERISTICS:

(A) LENGTH: 20 base pairs
(B) TYPE: nucleic add
(C) STRANDEDNESS: single
(D) TOPOLOGY: linear

(ii) MOLECULE TYPE: DNA (genomic)

(iii) HYPOTHETICAL NO

(iv) ANTI-SENSE. NO

(vi) ORIGINAL SOURCE:

(A) ORGANISM: Equus caballus

(vii) IMMEDIATE SOURCE:

(B) CLONE: 177-3

(xi) SEQUENCE DESCRIPTION: SEQ ID NO:31:


CTTCCTGGCA ATTGTAGGAC                    20


(2) INFORMATION FOR SEQ ID NO:32:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 177-3

(xi) SEQUENCE DESCRIPTION: SEQ ID NO:32:

AAGGCCCCCC ATTTCTTGCT                    20

(2) INFORMATION FOR SEQ ID NO:33:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL- NO

(iv) ANTI-SENSE: NO

(vi) ORIGINAL SOURCE:

(A) ORGANISM: Equus caballus

(vii) IMMEDIATE SOURCE:

(B) CLONE: 595-1

(xi) SEQUENCE DESCRIPTION: SEQ ID NO:33:

GAATATCAAT ATATATATAT                    20

(2) INFORMATION FOR SEQ ID NO:34:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-1

(xi) SEQUENCE DESCRIPTION: SEQ 10 NO :34:

TGTGTGTGTG TGTATTTGCT                                        20

(2) INFORMATION FOR SEQ ID NO:35:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL NO

(iv) ANTI-SENSE: NO

(vi) ORIGINAL SOURCE:

(A) ORGANISM: Equus caballus

(vii) IMMEDIATE SOURCE:

(B) CLONE: 595-1

(xi) SEQUENCE DESCRIPTION: SEQ ID NO:35:

AGCAAATACA CACACACACA                                        20

(2) INFORMATION -FOR SEQ ID NO:36:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-1

(xi) SEQUENCE DESCRIPTION: SEQ ID NO:36:

       **ATATATATAT ATTGATATTC**               **20**

(2) INFORMATION FOR SEQ ID NO:37:

  (i) SEQUENCE CHARACTERISTICS:

    (A) LENGTH: 20 base pairs
    (B) TYPE: nucleic acid
    (C) STRANDEDNESS: single
    (D) TOPOLOGY: linear

  (ii) MOLECULE TYPE: DNA (genomic)

  (iii) HYPOTHETICAL: NO

  (iv) ANTI-SENSE: NO

  (vi) ORIGINAL SOURCE:

    (A) ORGANISM: Equus caballus

  (vii) IMMEDIATE SOURCE:

    (B) CLONE: 007-3

  (xi) SEQUENCE DESCRIPTION: SEQ ID NO:37:

       **GCCATAATTA AGCCTGTATT**               **20**

(2) INFORMATION FOR SEQ ID NO:38:

  (i) SEQUENCE CHARACTERISTICS:

    (A) LENGTH: 20 base pairs
    (B) TYPE: nucleic acid
    (C) STRANDEDNESS: single
    (D) TOPOLOGY: linear

  (ii) MOLECULE TYPE: DNA (genomic)

  (iii) HYPOTHETICAL NO

  (iv) ANTI-SENSE: NO

  (vi) ORIGINAL SOURCE:

    (A) ORGANISM: Equus caballus

  (vii) IMMEDIATE SOURCE:

    (B) CLONE: 007-3

  (xi) SEQUENCE DESCRIPTION: SEQ ID NO:38:

GTTTGTTTTA AATTTTGTGA                    20

(2) INFORMATION FOR SEQ 10 NO:39

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL NO

    (iv) ANTI-SENSE: NO

    (vi) ORIGINAL SOURCE:

        (A) ORGANISM: Equus caballus

    (vii) IMMEDIATE SOURCE:

        (B) CLONE: 007.3

    (xi) SEQUENCE DESCRIPTION: SEQ 10 NO:39:

TCACAAAATT TAAAACAAAC                    20

(2) INFORMATION FOR SEQ ID NO:40:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 007-3

    (xi) SEQUENCE DESCRIPTION: SEQ ID NO:40:

AATACAGGCT TAATTATGGC                    20

(2) INFORMATION'FOR SEQ ID NO:41:

   (i) SEQUENCE CHARACTERISTICS:

      (A) LENGTH: 20 base pairs
      (B) TYPE: nucleic acid
      (C) STRANDEDNESS: single
      (D) TOPOLOGY: linear

   (ii) MOLECULE TYPE: DNA (genomic)

   (iii) HYPOTHETICAL: NO

   (iv) ANTI-SENSE: NO

   (vi) ORIGINAL SOURCE:

      (A) ORGANISM: Equus caballus

   (vii) IMMEDIATE SOURCE:

      (B) CLONE: 459-1

   (xi) SEQUENCE DESCRIPTION: SEQ ID NO:41:

        GTGTAGAGTA GTTCAAGGAC           20

(2) INFORMATION -FOR SEQ ID NO:42:

   (i) SEQUENCE CHARACTERISTICS:

      (A) LENGTH: 20 base pairs
      (B) TYPE: nucleic acid
      (C) STRANDEDNESS: single
      (D) TOPOLOGY: linear

   (ii) MOLECULE TYPE: DNA (genomic)

   (iii) HYPOTHETICAL: NO

   (iv) ANTI-SENSE: NO

   (vi) ORIGINAL SOURCE:

      (A) ORGANISM: Equus caballus

   (vii) IMMEDIATE SOURCE:

      (B) CLONE: 459-1

   (xi) SEQUENCE DESCRIPTION: SEQ ID NO:42:

        ATGTCTTATA CCTCCCTTTT           20

(2) INFORMATION FOR SEQ ID NO:43:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 459-1

(xi) SEQUENCE DESCRIPTION: SEQ ID NO:43:

          AAAAGGGAGG TATAAGACAT                    20

(2) INFORMATION FOR SEQ ID NO:44:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 459-1

(xi) SEQUENCE DESCRIPTION: SEQ ID NO:44:

          GTCCTTGAAC TACTCTACAC                    20

(2) INFORMATION FOR SEQ ID NO:45:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 085-1

(xi) SEQUENCE DESCRIPTION: SEQ ID NO:45:

GTGAACGGAG AGCAGGCCTT                                    20

(2) INFORMATION FOR SEQ ID NO:46:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 085-1

(xi) SEQUENCE DESCRIPTION: SEQ ID NO:46:

CCTGCTGAAG CCTCAGACCG                                    20

(2) INFORMATION FOR SEQ ID NO:47:

(i) SEQUENCE CHARACTERISTICS:

(A) LENGTH: 20 base pairs
(B) TYPE: nucleic acid

(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 085-1

(xi) SEQUENCE DESCRIPTION: SEQ ID NO:47:

CGGTCTGAGG CTTCAGCAGG                                                    20

(2) INFORMATION FOR SEQ ID NO:48:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 085-1

(xi) SEQUENCE DESCRIPTION: SEQ ID NO:48:

AAGGCCTGCT CTCCGTTCAC                                                    20

(2) INFORMATION FOR SEQ ID NO:49:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 007-2

(xi) SEQUENCE DESCRIPTION: SEQ ID NO:49:

CTGCTCTTTA GACTATGACC                                    20

(2) INFORMATION FOR SEQ ID NO:50:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 007-2

(xi) SEQUENCE DESCRIPTION: SEQ ID NO:50

TCAACCTTGC ATCATGAGCT                                    20

(2) INFORMATION FOR SEQ ID NO:51:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 007-2

(xi) SEQUENCE DESCRIPTION: SEQ ID NO:51:

        **AGCTCATGAT GCAAGGTTGA**        **20**

(2) INFORMATION FOR SEQ ID NO:52:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 007-2

    (xi) SEQUENCE DESCRIPTION: SEQ ID NO:52:

        **GGTCATAGTC TAAAGAGCAG**        **20**

(2) INFORMATION FOR SEQ ID NO:53:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 474-1

(xi) SEQUENCE DESCRIPTION: SEO ID NO:53:

       **TTTGAGCTGG GACCTCAGTC**        **20**

(2) INFORMATION FOR SEQ ID NO:54:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL- NO

    (iv) ANTI-SENSE: NO

    (vi) ORIGINAL SOURCE:

        (A) ORGANISM: Equus caballus

    (vii) IMMEDIATE SOURCE:

        (B) CLONE: 474-1

    (xi) SEQUENCE DESCRIPTION: SEQ ID NO:54:

       **TCTCCTGCCT TTAGACTCGA**        **20**

(2) INFORMATION FOR SEQ ID NO:55:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

(vi) ORIGINAL SOURCE:

    (A) ORGANISM: Equus caballus

(vii) IMMEDIATE SOURCE:

    (B) CLONE: 474-1

(xi) SEQUENCE DESCRIPTION: SEQ ID NO:55:

        TCGAGTCTAA AGGCAGGAGA            20

(2) INFORMATION FOR SEQ ID NO:56:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 474-1

    (xi) SEQUENCE DESCRIPTION: SEQ ID NO:56:

        GACTGAGGTC CCAGCTCAAA            20

(2) INFORMATION FOR SEQ ID NO:57:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

(A) ORGANISM: Equus caballus

(vii) IMMEDIATE SOURCE:

    (B) CLONE: 178-1

(xi) SEQUENCE DESCRIPTION: SEQ ID NO:57:

        GAACCTCTGG GCCGTGGATA           20

(2) INFORMATTON FOR SEQ ID NO:58:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 178-1

    (xi) SEQUENCE DESCRIPTION: SEQ ID NO:58:

        TTGTTCAGAA GCACAGGTGA           20

(2) INFORMATION FOR SEQ ID NO:59:

    (i) SEQUENCE CHARACTERISTICS:

        (A) LENGTH: 20 base pairs
        (B) TYPE: nucleic add
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

(vii) IMMEDIATE SOURCE:

    (B) CLONE: 178.1

(xi) SEQUENCE DESCRIPTION: SEQ ID NO:59:


          TCACCTGTGC TTCTGAACAA                       20


(2) INFORMATION FOR SEQ ID NO:60:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

    (B) CLONE: 178-1

(xi) SEQUENCE DESCRIPTION: SEQ ID NO.60:


          TATCCACGGC CCAGAGGTTC                       20


(2) INFORMATION FOR SEQ ID NO:61:

(i) SEQUENCE CHARACTERISTICS:

    (A) LENGTH: 20 base pairs
    (B) TYPE: nucleic acid
    (C) STRANDEDNESS: single
    (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Equus caballus

(vii) IMMEDIATE SOURCE:

(B) CLONE: 595-2

(xi) SEQUENCE DESCRIPTION: SEQ ID NO:61:

GTATTTGCTA GCTCTGGGAT                                                    20

(2) INFORMATION FOR SEQ ID NO:62:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-2

(xi) SEQUENCE DESCRIPTION: SEQ ID NO:62:

ATCCACTAAT GAGGGAAAAA                                                    20

(2) INFORMATION FOR SEQ ID NO:63:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 595-2

(xi) SEQUENCE DESCRIPTION: SEQ ID NO:63:

TTTTTCCCTC ATTAGTGGAT                    20

(2) INFORMATION FOR SEQ ID NO:64:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL NO

    (iv) ANTI-SENSE: NO

    (vi) ORIGINAL SOURCE:

        (A) ORGANISM: Equus caballus

    (vii) IMMEDIATE SOURCE:

        (B) CLONE: 595-2

    (xi) SEQUENCE DESCRIPTION: SEQ ID NO:64:

ATCCCAGAGC TAGCAAATAC                    20

(2) INFORMATION FOR SEQ ID NO:65:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 177-1

    (xi) SEQUENCE DESCRIPTION: SEQ ID NO:65:

GAAGTTGTGG GACAGATGTG                                    20

(2) INFORMATION FOR SEQ ID NO:66:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 177-1

    (xi) SEQUENCE DESCRIPTION: SEQ ID NO:66:

AGAGATGCAG CTCTAAGTGC                                    20

(2) INFORMATION FOR SEQ ID NO.67:

    (i) SEQUENCE CHARACTERISTICS:

        (A) LENGTH: 20 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Equus caballus

    (vii) IMMEDIATE SOURCE:

        (B) CLONE: 177-1

    (xi) SEQUENCE DESCRIPTION: SEQ ID NO:67:

GCACTTAGAG CTGCATCTCT 20

(2) INFORMATION FOR SEQ ID NO:68:

  (i) SEQUENCE CHARACTERISTICS:

    (A) LENGTH: 20 base pairs
    (B) TYPE: nucleic acid
    (C) STRANDEDNESS: single
    (D) TOPOLOGY: linear

  (ii) MOLECULE TYPE: DNA (genomic)

  (iii) HYPOTHETICAL: NO

  (iv) ANTI-SENSE: NO

  (vi) ORIGINAL SOURCE:

    (A) ORGANISM: Equus caballus

  (vii) IMMEDIATE SOURCE:

    (B) CLONE: 177-1

  (xi) SEQUENCE DESCRIPTION, SEQ ID NO:68:

CACATCTGTC CCACAACTTC 20

(2) INFORMATION FOR SEQ ID NO:69:

  (i) SEQUENCE CHARACTERISTICS:

    (A) LENGTH: 20 base pairs
    (B) TYPE: nucleic acid
    (C) STRANDEDNESS: single
    (D) TOPOLOGY: linear

  (ii) MOLECULE TYPE: DNA (genomic)

  (iii) HYPOTHETICAL: NO

  (iv) ANTI-SENSE: NO

  (vi) ORIGINAL SOURCE:

    (A) ORGANISM: Equus caballus

  (vii) IMMEDIATE SOURCE:

    (B) CLONE: 459-2

  (xi) SEQUENCE DESCRIPTION: SEQ ID NO:69:

CCATGAGGAA GCCTCCACAA 20

(2) INFORMATION FOR SEQ ID NO:70:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 459.2

(xi) SEQUENCE DESCRIPTION: SEQ ID NO:70:

GTCCCAATAG TCTGGGATTC 20

(2) INFORMATION FOR SEQ ID NO:71:

(i) SEQUENCE CHARACTERISTICS:

(A) LENGTH: 20 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 459-2

(xi) SEQUENCE DESCRIPTION: SEQ ID NO:71:

GAATCCCAGA CTATTGGGAC 20

(2) INFORMATION FOR SEQ ID NO:72:

(i) SEQUENCE CHARACTERISTICS:

(A) LENGTH: 20 base pairs
(B) TYPE: nucleic acid
(C) STRANDEDNESS: single
(D) TOPOLOGY- linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Equus caballus

(vii) IMMEDIATE SOURCE:

(B) CLONE: 459-2

(xi) SEQUENCE DESCRIPTION: SEQ ID NO:72:

TTGTGGAGGC TTCCTCATGG 20

(2) INFORMATION FOR SEQ ID NO:73:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE. NO

(vi) ORIGINAL SOURCE:

(A) ORGANISM: Homo sapiens

(vii) IMMEDIATE SOURCE:

(B) CLONE: IGKC 2p12

(xi) SEQUENCE DESCRIPTION: SEQ ID NO:73:

AAAGCAGACT ACGAGAAACA CAAA                          24

(2) INFORMATION FOR SEQ ID NO:74:

   (i) SEQUENCE CHARACTERISTICS:

      (A) LENGTH: 24 base pairs
      (B) TYPE: nucleic acid
      (C) STRANDEDNESS: single
      (D) TOPOLOGY: linear

   (ii) MOLECULE TYPE: DNA (genomic)

   (iii) HYPOTHETICAL: NO

   (iv) ANTI-SENSE: NO

   (vi) ORIGINAL SOURCE:

      (A) ORGANISM: Homo sapiens

   (vii) IMMEDIATE SOURCE:

      (B) CLONE: IGKC 2p12

   (xi) SEQUENCE DESCRIPTION: SEQ ID NO:74:

TCTACGCCTG CGAAGTCACC CATC                          24

(2) INFORMATION FOR SEQ ID NO:75:

   (i) SEQUENCE CHARACTERISTICS:

      (A) LENGTH: 24 base pairs
      (B) TYPE: nucleic acid
      (C) STRANDEDNESS: single
      (D) TOPOLOGY: linear

   (ii) MOLECULE TYPE: DNA (genomic)

   (iii) HYPOTHETICAL: NO

   (iv) ANTI-SENSE: NO

   (vi) ORIGINAL SOURCE:

      (A) ORGANISM: Homo sapiens

   (vii) IMMEDIATE SOURCE:

      (B) CLONE: IGKC 2p12

   (xi) SEQUENCE DESCRIPTION: SEQ ID NO:75:

GATGGGTGAC TTCGCAGGCG TAGA                24

(2) INFORMATION FOR SEQ ID NO:76:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: IGKC 2p12

(xi) SEQUENCE DESCRIPTION: SEQ ID NO:76:

TTTGTGTTTC TCGTAGTCTG CTTT                24

(2) INFORMATION FOR SEQ ID NO:77:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: ILIB 2q3-q21

(xi) SEQUENCE DESCRIPTION: SEQ ID NO:77:

CTCCTGCAAT TGACAGAGAG CTCC                                    24

(2) INFORMATION FOR SEQ ID NO:78:

    (i) SEQUENCE CHARACTERISTICS:

        (A) LENGTH: 24 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Homo sapiens

    (vii) IMMEDIATE SOURCE:

        (B) CLONE: ILIB 2q3-q21

    (xi) SEQUENCE DESCRIPTION: SEQ ID NO:78:

GAGGCAGAGA ACAGCACCCA AGGT                                    24

(2) INFORMATION FOR SEQ ID NO:79:

    (i) SEQUENCE CHARACTERISTICS:

        (A) LENGTH: 24 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Homo sapiens

    (vii) IMMEDIATE SOURCE:

        (B) CLONE: ILIB 2q3-q21

    (xi) SEQUENCE DESCRIPTION: SEQ ID NO:79:

ACCTTGGGTG CTGTTCTCTG CCTC                                    24

(2) INFORMATION FOR SEQ to NO:80:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: ILIB 2q3-q21

(xi) SEQUENCE DESCRIPTION: SEQ ID NO:80:

GGAGCTCTCT GTCAATTGCA GGAG                24

(2) INFORMATION FOR SEQ ID NO:81:

(i) SEQUENCE CHARACTERISTICS:

(A) LENGTH: 24 base pairs
(B) TYPE: nucleic add
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: LDLR 19p13.3

(xi) SEQUENCE DESCRIPTION: SEQ ID NO:81:

CTCCATCTCA AGCATCGATG TCAA                24

(2) INFORMATION FOR SEQ ID NO:82:

(i) SEQUENCE CHARACTERISTICS:

   (A) LENGTH: 24 base pairs
   (B) TYPE: nucleic acid
   (C) STRANDEDNESS: single
   (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

   (A) ORGANISM: Homo sapiens

(vii) IMMEDIATE SOURCE:

   (B) CLONE: LDLR 19p13.3

(xi) SEQUENCE DESCRIPTION: SEQ ID NO:82:

   GGGGGCAACC GGAAGACCAT CTTG                                    24

(2) INFORMATION FOR SEQ ID NO:83:

   (i) SEQUENCE CHARACTERISTICS:

      (A) LENGTH: 24 base pairs
      (B) TYPE: nucleic acid
      (C) STRANDEDNESS: single
      (D) TOPOLOGY: linear

   (ii) MOLECULE TYPE: DNA (genomic)

   (iii) HYPOTHETICAL: NO

   (iv) ANTI-SENSE: NO

   (vi) ORIGINAL SOURCE:

      (A) ORGANISM: Homo sapiens

   (vii) IMMEDIATE SOURCE:

      (B) CLONE: LDLR 19p13.3

   (xi) SEQUENCE DESCRIPTION: SEQ ID NO: 83:

      CAAGATGGTC TTCCGGTTGC CCCC                                 24

(2) INFORMATION FOR SEQ ID NO:84:

   (i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: LDLR 19p13.3

(xi) SEQUENCE DESCRIPTION: SEQ ID NO:84:

**TTGACATCGA TGCTTGAGAT GGAG** **24**

(2) INFORMATION FOR SEQ ID NO:85:

(i) SEQUENCE CHARACTERISTICS:

(A) LENGTH: 24 base pairs
(B) TYPE: nucleic add
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: MET-H 7q31

(xi) SEQUENCE DESCRIPTION: SEQ ID NO:85:

**GTTTGGTCTA AGTTGCTGAT TACC** **24**

(2) INFORMATION FOR SEQ ID NO:86:

(i) SEQUENCE CHARACTERISTICS:

(A) LENGTH: 24 base pairs
(B) TYPE: nucleic acid

(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Homo sapiens

(vii) IMMEDIATE SOURCE:

    (B) CLONE: MET-H 7q31

(xi) SEQUENCE DESCRIPTION: SEQ ID NO:86:

               GGATTTTTCT GACGATCTTT CAAC                        24

(2) INFORMATION FOR SEQ ID NO:87:

    (i) SEQUENCE CHARACTERISTICS:

        (A) LENGTH: 24 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Homo sapiens

    (vii) IMMEDIATE SOURCE:

        (B) CLONE: MET-H 7q31

    (xi) SEQUENCE DESCRIPTION: SEQ ID NO:87:

               GTTGAAAGAT CGTCAGAAAA ATCC                        24

(2) INFORMATION FOR SEQ ID NO:88:

    (i) SEQUENCE CHARACTERISTICS:

        (A) LENGTH: 24 base pairs
        (B) TYPE: nucleic acid
        (C) STRANDEDNESS: single
        (D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: MET-H 7q31

(xi) SEQUENCE DESCRIPTION: SEQ ID NO:88:

GGTAATCAGC AACTTAGACC AAAC                                   24

(2) INFORMATION FOR SEQ ID NO:89:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: PROC 2q13-q21

(xi) SEQUENCE DESCRIPTION: SEQ ID NO:89:

GCTGACAGCG GCCCACTGCA TGGA                                   24

(2) INFORMATION FOR SEQ ID NO:90:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: PROC 2q13-q21

(xi) SEQUENCE DESCRIPTION: SEQ ID NO:90:

GAGTCCAAGA AGCTCCTTGT CAGG

(2) INFORMATION FOR SEQ ID NO:91:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: PROC 2q13-q21

(xi) SEQUENCE DESCRIPTION: SEQ ID NO:91:

CCTGACAAGG AGCTTCTTGG ACTC

(2) INFORMATION FOR SEQ ID NO:92:

(i) SEQUENCE CHARACTERISTICS:

(A) LENGTH: 24 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

    (A) ORGANISM: Homo sapiens

(vii) IMMEDIATE SOURCE:

    (B) CLONE: PROC 2q13-q21

(xi) SEQUENCE DESCRIPTION: SEQ ID NO:92:

**TCCATGCAGT GGGCCGCTGT CAGC**          **24**

(2) INFORMATION FOR SEQ ID NO-93:

    (i) SEQUENCE CHARACTERISTICS:

        (A) LENGTH: 24 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:

        (A) ORGANISM: Homo sapiens

    (vii) IMMEDIATE SOURCE:

        (B) CLONE: MET-H 7q31

    (xi) SEQUENCE DESCRIPTION: SEQ ID NO:93:

**CATCCATGTA GGAGAGCCTT AGTC**          **24**

(2) INFORMATION FOR SEQ ID NO:94:

    (i) SEQUENCE CHARACTERISTICS:

        (A) LENGTH: 26 base pairs
        (B) TYPE: nucleic acid
        (C) STRANDEDNESS: single
        (D) TOPOLOGY: linear

    (ii) MOLECULE TYPE: DNA (genomic)

    (iii) HYPOTHETICAL: NO

    (iv) ANTI-SENSE: NO

(vi) ORIGINAL SOURCE:

(A) ORGANISM: Homo sapiens

(vii) IMMEDIATE SOURCE:

(B) CLONE: MET-H 7q31

(xi) SEQUENCE DESCRIPTION: SEQ ID NO:94:

**CCATTTTTGT GTCTTCTAGT CTAAGG** 26

(2) INFORMATION FOR SEQ ID NO:95:

(i) SEQUENCE CHARACTERISTICS:

(A) LENGTH: 23 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(vi) ORIGINAL SOURCE:

(A) ORGANISM: Homo sapiens

(vii) IMMEDIATE SOURCE:

(B) CLONE: MET-H 7q31

(xi) SEQUENCE DESCRIPTION: SEQ ID NO:95:

**TTGAAAGATC GTCAGAAAAA TCC** 23